# EUROPEAN PATENT APPLICATION

(11) **EP 2 636 413 A1**
(43) Date of publication of application: **11.09.2013**
(21) Application number: 12305283.9
(22) Date of filing: 09.03.2012
(51) Int. Cl.: A61K 39/155, C12N 7/00, G01N 33/569

(54) **Chimeric virus-like particles (VLP) containing functional hMPV proteins**

(71) Applicant: Ecole Normale Superieure De Lyon, 69007 Lyon (FR); UCBL Université Claude Bernard de Lyon 1, 69622 Villeurbanne Cedex (FR); Université Laval, Québec, Québec G1K 7P4 (CA)
(72) Inventor: Boivin, Guy, Québec QC G1V1E7 (CA); Cosset, François Loïc, 69004 Lyon (FR); Levy, Camille, 69007 Lyon (FR); Lavillette, Dimitri, 69007 Lyon (FR); Hamelin, Marie-Eve, Québec QC G2B0K6 (CA); Aerts, Laetitia, Québec QC G1S3Y1 (CA)
(74) Representative: Colombet, Alain André

(57) **Abstract**

The invention relates to the generation and the use of chimeric pseudo-particles VLPs containing hMPV glycoproteins assembled onto retroviral core particles. These particles may be highly infectious and constitute a valid model of hMPV virion. The invention also relates to the development of a vaccine based on such particles. The VLPs exhibit the hMPV F protein, possibly associated with the G protein. The retroviral core particle may be made from Gag protein, possibly with Pol.

## Description

The invention relates to the generation and the use of chimeric pseudo-particles containing hMPV glycoproteins assembled onto retroviral core particles. These particles may be highly infectious and constitute a valid model of hMPV virion. The invention also relates to the development of a vaccine based on such particles.

### Background of invention

Human metapneumovirus (hMPV) is a newly described virus member of the Paramyxoviridae family, assigned to the genus Metapneumovirus in the subfamily of Pneumovirinae (44). hMPV is related to respiratory syncytial virus (RSV), which is the most significant respiratory pathogen of infancy and early childhood. Epidemiologic studies showed that hMPV is associated with significant morbidity in infants and other high-risk populations, such as immunocompromised patients and individuals with underlying conditions, including prematurity, asthma, and cardiopulmonary disease (6, 30, 46, 47). Hospitalization rates due to hMPV infection are comparable to those caused by other common respiratory viruses such as RSV, parainfluenza virus (PIV), or influenza virus.

The hMPV genome consists of a single negative strand of RNA of approximately 13 kb, containing eight genes that are presumed to encode nine different proteins (4, 43). hMPV isolates can be separated into two major lineages (A and B) and at least four subgroups (5, 31). Nucleotide and amino acid sequence identities between hMPV lineages A and B are 80 and 90 %, respectively (4). hMPV displays 3 surface glycoproteins : F, responsible of membrane fusion, G involved in cell attachment and SH, a small hydrophobic protein. The F glycoprotein is highly conserved between the two major lineages. Moreover, the antibodies induced against the F protein correlate with protection in animal models indicating that F induces a humoral immune response (37, 41).

As hMPV has a major impact on human health, safe and effective vaccines could decrease the burden of diseases associated with this new pathogen. Since the discovery of hMPV (44), several studies have focused on the development of a hMPV vaccine. Different strategies have been investigated, such as inactivated virus, live-attenuated virus, recombinant proteins, or DNA encoding viral proteins. A major challenge is the difficulty to induce strong and long-lasting immune responses through the development of a sufficiently safe vaccine especially for treatment of high-risk population (immunocompromised patients or infants). Indeed, recombinant proteins do not always induce potent immune responses and live-attenuated viruses present potential risks. Thus, alternative vaccine candidates for hMPV are required to induce sustained immune responses and, specifically, high-titer neutralizing antibodies.

An approach to induce potent humoral responses relies in the development of VLP-based vaccines (34). Indeed, VLPs mimic the overall structure of the surface of virus particles and can be used as carriers for foreign antigens (3). Several attempts to develop VLPs based vaccines have been performed, however with mitigated success.

For non-enveloped viruses, such as papillomavirus, neutralizing antibodies have been successfully generated by VLPs (19). For enveloped viruses, formation of VLPs is a more complex process, since it requires the expression of multiple proteins in cell culture systems.

Homologous VLPs have been recently developed for Chikungunya virus (1) or SARS virus (24); however, it appears to be inefficient for most other viruses.

Alternatively, as shown for influenza (39, 40) or HCV (15), retrovirus-derived VLPs could represent a flexible and efficient platform for developing vaccines. Indeed, such VLPs are formed by the sole expression of the retroviral Gag protein, on which a wide array of heterologous viral envelope proteins can be functionally displayed (9). For influenza or HCV, VLPs have recently been shown to induce potent immune responses. To be promising, such chimeric VLP model requires at least that the target immunogens be displayed correctly at the surface of the VLPs and that these immunogens are able to induce an immunogenic response and preferably a protective immune response, which require at least a correct conformation for the immunogen.

VLPs have been also recently reported for the development of RSV vaccines (26, 28, 33). However, production of such VLPs, based on the expression of RSV structural proteins alone, was inefficient (26). In contrast, alternative VLPs based on assembly of RSV glycoproteins onto heterologous viral core derived from Newcastle disease Virus (26, 28) or from influenza virus (33), were efficiently produced. These VLPs displaying RSV F and/or G induced neutralizing antibodies and protected mice against RSV infection.

In addition, specific and quantitative assembly of glycoproteins on heterologous core is not always efficient as it often requires modification of the cytoplasmic tail or the transmembrane of the glycoproteins to interact with or to be sterically compatible with the core proteins (23). Envelope glycoproteins from measles virus (13) or from Nipah virus (25), which are other paramyxoviruses, can be incorporated on retroviral cores but this required truncation of their cytoplasmic tails. Indeed, efficient and functional co-display of their H or G proteins, responsible for virus-receptor binding, and of their F fusion glycoproteins at a specific ratio and in a specific conformation was necessary to allow efficient virus-cell binding and fusion (13).

Recent studies showed that the hMPV-F protein interact with an integrin (8), whereas the hMPV-G protein binds glycosaminoglycans and is probably involved in the preliminary attachment of the virus to the cell (42). In addition, it has been shown that the G protein does not harbor virus-cell fusion determinants (43). Previous studies have also shown that anti-G antibodies do not neutralize hMPV (27, 35) whereas F was shown to be the major protective antigen (37, 41). Consequently, several vaccine strategies have been based on the use of recombinant soluble F protein, which proved to induce an efficient immune response (8, 21, 22).

Naturally hMPV-infected patients exhibit a transient production of hMPV specific antibodies (29), which was corroborated in experimentally infected in macaques that showed a decrease over time of the protective immunity induced by hMPV infection (45).

Cross-protection was detected when animals were vaccinated with hMPV from one lineage and challenged with another hMPV lineage (37, 38, 45). In contrast, soluble F protein used as immunogen only induced partial cross-protection between the two main lineages of hMPV (21). In addition, in the context of alphavirus replicon-based vaccine expressing an hMPV F from the A lineage, cross-neutralization against the hMPV from the B lineage was also very low (27).

### Summary of invention

Here, we succeeded in generating chimeric retroviral-based VLPs displaying hMPV glycoproteins. These VLPs displays efficiently the hMPV F glycoprotein, possibly associated with other hMPV proteins such as the G protein. We demonstrate that immunization with these particles stimulated robust neutralizing antibody response in mice. Furthermore, they induced protection against homologous and heterologous hMPV challenge. Chimeric VLPs appear to be the good strategy to develop a safe and efficient vaccine against hMPV, especially for high-risk population (immunocompromised patients or infants). Infectious VLPs have also been generated which could be used for vaccination, for diagnosis or for screening.

One object of the invention is a chimeric virus-like particle, comprising a retrovirus Gag protein, and a human metapneumovirus (hMPV) F protein.

Another object of the invention is an immunogenic or vaccine composition comprising:
- A chimeric virus-like particle according to the invention and a pharmaceutically acceptable carrier or vehicle, or
- an expression vector or expression vectors in a pharmaceutically acceptable carrier or vehicle able to produce *in vivo* a chimeric virus-like particle according to the invention.

Another object of the invention is an expression vector or a set of expression vectors usable in the immunogenic or vaccine composition of the invention, comprising:
- a first nucleic acid sequence comprising a cDNA encoding a retrovirus Gag protein ;
- a second nucleic acid sequence comprising a cDNA encoding a F protein of a human metapneumovirus (hMPV);
- wherein said first and second nucleic acid sequences are contained within the same vector or different vectors;
- and regulatory elements allowing in a human being expression of the cDNAs to produce F from hMPV and the retrovirus Gag protein, and the proteins to form virus-like particles.

Another object of the invention is a method for *ex vivo* producing a chimeric virus-like particle, possibly infectious, according to the invention, comprising the steps of:
-- providing a first nucleic acid sequence comprising a cDNA encoding a retrovirus Gag protein ;
   - providing a second nucleic acid sequence comprising a cDNA encoding a F protein of hMPV;
   - transfecting host cells with said nucleic acid sequences and maintaining the transfected cells in culture for sufficient time to allow expression of the cDNAs to produce structural proteins from hMPV and retrovirus; and allowing the structural proteins to form virus-like particles,
   - possibly treating the obtained virus-like particles with an agent that cleaves F into F1 and F2.

Another object of the invention is a method of *ex vivo* screening or identification of molecules capable of interfering with human metapneumovirus (hMPV) entry in cells comprising comparison of the level of cell infection by an infectious chimeric virus-like particle according to the invention in the presence or the absence of a candidate molecule.

Another object of the invention is a method of *in vitro* diagnosis of human metapneumovirus (hMPV) infection in a patient, comprising detecting immune complexes formed by interaction of anti-HMPV antibodies likely to be present in a biological sample of the patient with chimeric virus-like particle according to the invention.

Another object of the invention is a method of *in vitro* diagnosis human metapneumovirus (hMPV) infection in a patient, comprising detecting an inhibitory effect of anti-hMPV antibodies likely to be present in a biological sample of the patient, on the infection of a permissive cell by infectious chimeric virus-like particle according to the invention.

Another object of the invention is a transformed host cell that contains:
- a first nucleic acid sequence comprising a cDNA a retrovirus Gag protein;
- a second nucleic acid sequence comprising a cDNA encoding a human metapneumovirus (hMPV) F protein,
- possibly a third nucleic acid sequence comprising a packaging competent retroviral-derived genome.

Still another object of the invention is a method of *ex vivo* screening or identification of molecules capable of interfering with human metapneumovirus (hMPV) entry in cells comprising comparing the level of fusion of transformed host cell, as defined, to a target host cell, in the presence or the absence of a candidate molecule.

Other objects of the invention will be apparent from the following description.

### Detailed description

Development of vaccines that prevent serious hMPV respiratory tract disease in young children, immuno-compromised individuals and the elderly is required. In this study, we used a new approach to develop a hMPV vaccine or immunogenic composition based on retroviral VLPs. This new approach relies on the incorporation of the hMPV glycoproteins from either of the two main lineages onto retroviral cores. The validity of these VLPs displaying the F or the F and G hMPV glycoproteins as a vaccine or as an immunogenic composition was evaluated *in vivo* in mice. We show that the hMPV-VLPs stimulated cross-neutralizing humoral immune responses. Immunization of mice with these particles resulted in protection against hMPV infection in the lungs, the primary organ of infection and replication.

We showed that both F and G hMPV glycoproteins can be incorporated on heterologous retroviral core particles. Unmodified hMPV-F glycoproteins were readily and functionally incorporated on MLV particles, which resulted in infectious particles. This indicated that the hMPV-F glycoprotein supported both receptor binding and fusion functions. In contrast, VLPs displaying hMPV-G proteins alone did not result in infectious particles. In the context of our VLPs, the co-incorporation of G together with F is possible, despite the fact that the presence of G slightly decreased infectious titers, which could explain the slightly decreased incorporation F on the retroviral core when both glycoproteins were displayed.

Here, we showed that hMPV-VLPs based on retroviral cores are efficient immunogens and elicit robust, cross-reactive humoral responses against hMPV. In the case of hMPV, the incorporation of the F protein on the VLPs alone was sufficient to induce neutralizing antibodies whereas co-incorporation of F and G proteins did not improve the yields of neutralizing antibodies.

Of note, hMPV-specific antibody titers induced by our F-VLPs or F/G-VLPs persisted over time.

We showed that hMPV-VLPs displaying F from either lineage A (C-85473) or B (Can98-75) of hMPV induced cross-neutralizing antibodies and cross-protection. Thus, our VPLs displaying hMPV F protein most probably induced potent cross-neutralizing antibody responses because the display on retroviral core particles likely presents the F glycoprotein in its native conformation, in contrast to soluble F protein which lacks specific determinants important for its correct conformation.

Importantly, when mice vaccinated with hMPV-VLPs were challenged with hMPV, they survived the challenge and virus replication was strongly reduced in the lungs. Moreover, although hMPV Can98-75 was very virulent and although residual virus was still detected in the lungs after 5 days post-challenge, most of vaccinated animals survived this challenge, in contrast to naïve mice that all died following infection. Finally, in agreement with the results of neutralization assays, no difference was observed between the use of the F protein of either lineage A or the B, which demonstrated cross-protection induced by our VLPs. Similarly to the results of neutralization assays, co-presentation of G and F proteins on the VLPs did not appear to influence the protection *in vivo.* VLPs incorporating F from any lineage seemed therefore to be sufficient to confer cross-protection against hMPV challenge.

The terms "*vector*"*, "cloning vector*" and *"expression vecto*r*"* mean the vehicle by which a DNA or RNA sequence (e.g. a foreign gene) can be introduced into a host cell, so as to transform the host and promote expression (e.g. transcription and translation) of the introduced sequence. Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "*DNA construct*"*.* A common type of vector is a "*plasmid*"*,* which generally is a self-contained molecule of doublestranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts.

A *"coding sequence*" or a sequence "*encoding*" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme.

The term "*transfection*" means the introduction of a foreign nucleic acid (DNA, cDNA or RNA) into a cell so that the host cell will express the introduced gene or sequence to produce a desired substance, typically a protein coded by the introduced gene or sequence. The introduced gene may include regulatory or control sequences, such as start, stop, promoter, signal, secretion, or other sequences used by a cell's genetic machinery. In particular, the gene, especially the cDNA, according to the invention, comprises a start codon, a promoter, possibly an enhancer, a polyA and a stop codon. A host cell that receives and expresses introduced DNA or RNA has been "*transformed*"*.* A preferred promoter is the immediate early cytomegalovirus promoter (CMV or CMV-IE). Other suitable promoters include: Elongation factor-1 alpha (EF-1alpha), Spleen Focus Forming Virus promoter (SFFV), human Ubiquitin promoter, human Phosphoglycerate kinase (PGK). Suitable enhancers include: CMV enhancer, SV40 enhancer, beta globin HS1 to HS4 sequences. Suitable Poly A include: SV 40 poly A, Thymidine Kinase Poly A, Rabbit beta-globin Poly A.

The term *"host cell*" means any cell of any organism that is selected, modified, transformed, grown, or used or manipulated in any way, for the production of a substance by the cell, for example the expression by the cell of a gene, a DNA sequence, a protein, a virion. In the context of the invention, the host cell is a mammalian cell. In particular, the host cell may be selected from the group consisting of mononuclear macrophages, monocytes or dendritic cells, endothelial cells, fibroblasts, neurone/neuroblastoma cells, hepatocyte/hepatoma cells. Suitable host cells include 293T human embryo kidney cells (ATCC CRL-1573), Hela (human epithelial adenocarcinoma cell line) (CCL-2), PS, LLC-MK2 (CCL-7), Hep2 (Epidermoid carcinoma) (ATCC CCL-23), TE671 human rhabdomyosarcoma (CRL-8805), Vero (African green monkey kidney) (CCL-81 or CRL-1587), As used herein, the term *"permissive cell"* is meant for a cell that is permissive for hMPV infection. Examples of permissive cells include the above-mentioned mammalian mononuclear macrophages, monocytes or dendritic cells, endothelial cells, fibroblasts, neurone/neuroblastoma cells, hepatocyte/hepatoma cells.

As used herein, the term *"permissive cell"* is meant for a cell that is permissive for hMPV infection. Examples of permissive cells include the above-mentioned mammalian mononuclear macrophages, monocytes or dendritic cells, endothelial cells, fibroblasts, neurone/neuroblastoma cells, hepatocyte/hepatoma cells.

The term *"variant"* refers to the homologous polynucleotide sequences and corresponding amino acid sequences found in the different hMPV strains owing to hMPV variability.

The *"core protein from a retrovirus"* refers to proteins encoded by the gag and/or pol genes. The gag gene encodes a polyprotein which is further processed by the retroviral protease into structural proteins that form the core. The pol gene encodes the retroviral protease, reverse-transcriptase, and integrase. Expression of the gag gene alone is sufficient to induce the release of core particles; yet such particles are not infectious as a result of absence of the pol gene-encoded retroviral replication enzymes. Therefore, the "core protein from a retrovirus" may denote proteins encoded by the *gag* and *pol* genes or by the *gag* gene alone.

The term *"chimeric virus-like particle"* or *"chimeric pseudoparticle"* or *"VLP"* as used herein refers to non-naturally occurring viral particles based on or made of the retrovirus Gag protein. The Gag protein is assembled to form a retroviral particle, with the F and/or G proteins of hMPV exposed on their surface. By itself, the Gag VLP is not infectious. The VLPs may include other retroviral enzymatic proteins, such as Pol, and harbor an expression vector resulting in infectious VLP for target cells. The infectious VLPs exposing hMPV F of the invention are highly infectious for a target cells. Such particles may be readily produced by one skilled in genetic engineering techniques. One can for instance refer to EP 1 201 750 that describes production of synthetic retroviral particles expressing an antigen for modulating an immune response. One may also refer to EP 2 338 984.

In the context of the invention, the term "*infectious*" is used to describe the capacity of the particles of the invention to complete the initial steps of viral cycle that lead to cell entry. However, upon interaction with the host cell, depending on their composition, the virus-like particles of the invention may or may not produce progeny viruses.

The term "gene" means any coding nucleic acid molecule. The term "gene" includes not only genomic DNA, but also cDNA, synthetic DNA, RNA, etc.

The term "protein" is used interchangeably with "polypeptide" and designates any molecule comprising an amino acid or an amino acid chain, optionally modified, glycosylated, etc.

The term *"an envelope protein of hMPV*" denotes the F or the G glycoprotein of hMPV, or a mutant thereof.

By a *"F glycoprotein"* or *"F protein*" is meant a F protein from any genotype, subtype and variant of hMPV strains. It is preferably the whole protein, comprising the cytoplasmic tail, the transmembrane domain and the extracellular domain. In an embodiment, the F protein comprises the transmembrane domain and the extracellular domain. The F protein according to the invention also encompasses a truncated F protein comprising the transmembrane domain, possibly the cytoplasmic tail, and only part of the extracellular domain which is still able to elicit an immune response, preferably similar or identical to the whole protein, able to confer immunization capability to the VLPs according to the invention and preferably protective capability for use in a vaccine composition.

By an *"G glycoprotein*" or *"G protein*" is meant a G protein from any genotype subtype and variant of hMPV strains.. It is preferably the whole protein, comprising the cytoplasmic quail, the transmembrane domain and the extracellular domain. In an embodiment, the G protein comprises the transmembrane domain and the extracellular domain. It also encompasses a truncated protein comprising the transmembrane domain, possibly the cytoplasmic quail, and only part of the extracellular domain which keeps the function of the whole protein.

Group B strain C-85473 has been described in ref. 18. Sequences are disclosed as:
- SEQ ID NO: 9 = F nucleic acid sequence
- SEQ ID NO: 10 = G nucleic acid sequence
- SEQ ID NO:11 = F amino acid sequence
- SEQ ID NO: 12 = G amino acid sequence

Whole genome of hMPV group A Can98-75 was published in reference (4). Accession number in GenBank is AY297748.1. Sequences of the F and G genes and proteins are given. Sequences are disclosed herein as:
- SEQ ID NO: 13 = F nucleic acid sequence
- SEQ ID NO: 14 = G nucleic acid sequence
- SEQ ID NO:15 = F amino acid sequence
- SEQ ID NO: 16 = G amino acid sequence

Other hMPV strains are known as a basis for F and/or G proteins, i.e. those of sub-group A NL00-1 and CAN97-83, and one of sub-group B CAN97-82.

Identity at the amino acid level for the F protein between the different genotypes is about 96%. The F protein used in the present invention may be defined as encompassing any variant amino acid sequence having at least 80%, in particular at least 85%, preferably at least 90%, 91%, 92%, 93%, 94%, 95%, 96%, 97%, 98% or 99% identity with SEQ ID NO: 9 or 13. Identity at the nucleotide level may be defined as encompassing any nucleic acid coding for such a variant amino acid sequence.

The term "*mutant*" or "*mutation*" is meant for alteration of the DNA sequence that result in a modification of the amino acid sequence of native F or G proteins. Such a modification can be for instance the substitution and/or deletion of one or more amino acids. Mutants notably include fragments of native F and G proteins. Variants are particular examples of naturally occurring mutants. Mutants are more particularly contemplated as useful for identifying the structural elements of F and/or G proteins necessary for maintaining cell infectivity or for increasing F and/or G immunogenicity for vaccination purposes.

In the context of the invention, the terms *"native"* or *"unmodified"* are indifferently used to describe a wild-type, full-length protein.

By *"retrovirus"* is meant a virus whose genome consists of a RNA molecule and that comprises a reverse-transcriptase, *i.e.* a member of the Retroviridae family. Retroviruses are divided into Oncovirus, Lentivirus and Spumavirus. Preferably said retrovirus is an oncovirus, *e.g.* MLV, ALV, RSV, or MPMV, a lentivirus, *e.g.* HIV-1, HIV-2, SIV, EIAV, or CAEV, or a spumavirus such as HFV. Genomes of these retroviruses are readily available in databanks.

In the context of the invention *"a nucleic sequence comprising a packaging competent retrovirus-derived genome"* is intended for a sequence that comprises the retroviral nucleic acid sequences known as "cis-acting" sequences. These include the Long Terminal Repeats (LTRs) for the control of transcription and integration, the psi sequence necessary for encapsidation, and the Primer Binding site (PBS) and polypurine track (PPT) sequences necessary for reverse transcription of the retroviral genome. Advantageously, said nucleic acid sequence comprising a packaging competent retrovirus-derived genome further comprises a transgene.

Said retroviral genome may be replication-defective or replication-competent, in the absence of any trans-complementing function. A replication-competent genome would further comprise the gag, pol, and env retroviral genes. In a replication-defective genome, the viral genes gag, pol, and env are deleted. However, assembly of viral pseudo-particles may be achieved by providing another vector that comprises gag, pol and env but that is defective for the "cis" sequences. Their expression allows the encapsidation of the transgene, excluding the genes necessary for the multiplication of the viral genome and for the formation of complete viral particles.

As used herein, the term *"transgene"* designates the gene that is expressed in the target cell upon infection by the particles of the invention.

Examples of transgenes include a gene encoding a molecule of therapeutic interest, a marker gene, a gene coding for an immune modulator, an antigen, or a suicide gene.

A "*marker gene*" denotes a gene whose expression is detectable. For instance marker gene expression can generate a detectable signal, such as a fluorescence emission, a chromogenic reaction, or confer a growth advantage to the cells wherein it is expressed (antibiotic resistance genes).

An *"immune modulator*" refers to the product of a gene that modifies the activity of the immune system of a subject *in vivo.* Examples of immune modulators include cytokines, (e.g. interleukins, interferons, or haematopoietic colony stimulating factors), chemokines, and the like. Expression of an immune modulator by transformed cells may change the cellular environment and alter differentiation of immune cells and thus modify the type and the strength of immune response elicited against a given immunogen.

An "*immunogen*" refers to a molecule, such as a peptide, a polypeptide or a protein, against which an immune response is sought. Said antigen may be for instance a tumor, a bacterial, a pathogenic, a proteic, or a viral antigen.

The "*immunogenic composition"* notion covers any composition which, once administered to the target species, induces an immune response directed against the hMPV. The immune response may not be protective. The term "*vaccine*" or *"vaccine composition"* is understood to mean a composition able to induce an effective protection.

A "*pharmaceutically acceptable carrier*" or "*pharmaceutically acceptable vehicle"* refers to any vehicle wherein the immunogenic composition or the vaccine composition according to the invention may be formulated. It includes a saline solution such as phosphate buffer saline. In general, a diluent or carrier is selected on the basis of the mode and route of administration, and standard pharmaceutical practice.

In the context of the present application, "*vaccination*" is intended for prophylactic or therapeutic vaccination. "*Therapeutic vaccination*" is meant for vaccination of a patient with hMPV infection.

According to the invention, the term "*subject*" or "*patient*" is meant for any mammal, human or animal, especially human, likely to be infected with hMPV.

### Production of chimeric virus-like particles

The inventors have generated chimeric virus-like particles that contain functional, and more particularly unmodified, hMPV glycoproteins assembled onto retroviral core particles.

The invention thus provides a method for producing chimeric virus-like particles *ex vivo* comprising the steps of:
- providing a first nucleic acid sequence comprising a cDNA encoding a retrovirus core protein ;
- providing a second nucleic acid sequence comprising a cDNA encoding a F protein, a G protein or F and G proteins of hMPV;
- transfecting host cells with said nucleic acid sequences and maintaining the transfected cells in culture for sufficient time to allow expression of the cDNAs to produce structural proteins from hMPV and retrovirus; and allowing the structural proteins to form virus-like particles.

The invention further provides a method for producing chimeric virus-like particles *in vivo,* which method comprises the steps of :
- providing a first nucleic acid sequence comprising a cDNA encoding a retrovirus core protein ;
- providing a second nucleic acid sequence comprising a cDNA encoding a F protein, a G protein or F and G proteins of hMPV;
- transfecting cells of a subject *in vivo* with said nucleic acid sequences, to allow expression of the cDNAs to produce structural proteins from hMPV and retrovirus; and to allow the structural proteins to form virus-like particles.

In an embodiment, the *ex vivo* or the *in vivo* method comprise the step of providing a second nucleic acid sequence comprising a cDNA encoding a F protein as sole protein of hMPV. This sequence preferably codes for the whole protein with its transmembrane domain. The invention also encompasses the use of a nucleic acid sequence coding for the transmembrane domain and a fragment or part of the extracellular domain as explained before.

In another embodiment, the *ex vivo* or the *in vivo* method also comprises the step of:
- providing a third nucleic acid sequence comprising a packaging competent retrovirus-derived genome.

In this embodiment, the method thus comprises the steps of:
- providing a first nucleic acid sequence comprising a cDNA encoding a core protein from said retrovirus;
- providing a second nucleic acid sequence comprising a cDNA encoding a F protein, a G protein or F and G proteins of hMPV;
- providing a third nucleic acid sequence comprising a packaging competent retrovirus-derived genome;
- transfecting host cells with said nucleic acid sequences and maintaining the transfected cells in culture for sufficient time to allow expression of the cDNAs to produce structural proteins from hMPV and retrovirus; and allowing the structural proteins to form virus-like particles.

In an embodiment, the second nucleic acid sequence comprises a cDNA encoding the F protein of hMPV. In an embodiment, the second nucleic acid sequence comprises a cDNA encoding the F and G proteins of hMPV. Alternatively, there are two nucleic acid sequences, one comprising a cDNA encoding the hMPV F protein, the other comprising a cDNA encoding the hMPV G protein.

The second nucleic acid sequence may comprise a cDNA encoding a F protein from sub-group A hMPV, from sub-group B hMPV or from sub-group A hMPV and sub-group B hMPV.

The second nucleic acid sequence may comprise a cDNA encoding F and G proteins from sub-group A hMPV, from sub-group B hMPV or from sub-group A hMPV and sub-group B hMPV.

In an embodiment of the *ex vivo* method, the obtained virus-like particles are treated with an agent that cleaves F into F1 and F2. In an embodiment, said agent is a protease that is able to makes this cleavage. In a preferred embodiment, said agent or protease is trypsine.

For the in vivo method, such a cleavage may be ensured by the proteases present in the subject.

In an embodiment, said cDNA encoding a retrovirus core protein contain the *gag* and pol genes, or expresses the Gag and Pol proteins. In a preferred embodiment, said cDNA encoding a retrovirus core protein contain the *gag* gene, or expresses the Gag protein. Infectious VLPs are produced.

In an embodiment, the obtained virus-like particles are treated with an agent that cleaves F into F1 and F2. In an embodiment, said agent is trypsine.

Said cDNA encoding the core proteins from said retrovirus may contain the *gag* and *pol* genes, or alternatively the *gag* gene alone.

According to the invention, the first nucleic acid sequence comprises a cDNA encoding a core protein, especially a Gag and/or Pol, preferably a Gag protein, of MLV, ALV, RSV, MPMV, HIV-1, HIV-2, SIV, EIAV, CAEV, or HFV, especially MLV.

For the purpose of transfection, said first, second and third nucleic acid sequences may be carried on a same vector, or on two or three separated vectors.

In particular, plasmoviruses, adenoretroviruses, replicating pseudo-viruses, and replicating virus vectors derived from measle virus, are examples of vectors suitable for carrying the above-mentioned sequences. A plasmovirus vaccine consists in such a plasmid DNA preparation, that allow expression of the pseudo-particles after administration in an patient in order to elicit a immune response against hMPV. Administration of such a plasmovirus vaccine being achieved for preventive vaccination into people at risk for hMPV-induced disease or for therapeutic vaccination into hMPV-infected patients. Adenoretroviruses consist in an alternative way to provide the above-mentioned nucleic acid sequences encoding pseudo-particles. In this case, it is possible to design three independent adenoretroviruses, *i.e.* recombinant adenoviruses, that encode the two or three nucleic acid sequences mentioned above (retroviral core and genome and hMPV glycoproteins), or, alternatively, it is also possible to design a single adenoretrovirus, derived from "guttless" recombinant adenoviruses, that contains the different nucleic acid sequences. Such adenoretroviruses can be administered to patient as for plasmoviruses, in order to elicit an anti-hMPV immune response. Replicating pseudo-retroviruses are another alternative possibility to express all the above-mentioned nucleic acid sequences encoding the pseudo-particles. Such structures are in fact pseudo-particles whose genome is engineered to allow, following infection, its propagation into cells of an inoculated patient, thereby inducing the production of further replicating pseudo-particles. In this case the genome of a retrovirus is modified so as to express the hMPV F and/or G glycoproteins in place of the retroviral Env gene (encoding the retroviral glycoproteins). The genes encoding the retroviral core proteins are left unchanged. Furthermore an additional gene, encoding a marker gene or an immunomodulator, for example, can be expressed from this genome. Measle virus (MV) vectors are derived from vaccinal strains of MV and harbour a replication-competent MV genome containing an additional gene encoding an immunogen. Replication of such recombinant MV elicits a strong immune response against this immunogen as shown, for example, in mice inoculated with MV vectors expressing HIV-1 antigens that induce strong neutralizing antibody and cellular immune response to HIV. In the context of this invention, MV-derived vectors harbour, as additional gene, a construct that express the hMPV F and/or G, preferably F, protein as well as the retroviral core proteins encoded by the gag and pol gene, or, alternatively, the gag gene only. Replication of this MV-based vector can induce the sustained secretion of pseudo-particles that, in turn, can elicit a vigorous immune response against hMPV proteins.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator.

In accordance with the present invention there may be employed conventional molecular biology, microbiology, and recombinant DNA techniques within the skill of the art. Such techniques are explained fully in the literature. See, e.g., Sambrook et al., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et *al.,* 1994.

In particular, the vectors of the invention may be introduced into the target cell by means of any technique known for the delivery of nucleic acids to the nucleus of cells, either in culture, *ex vivo,* or *in vivo.*

Introduction of the nucleic acid sequences may be performed by any standard method well known by one skilled in the art, e.g. transfection, electroporation, microinjection, transduction, cell fusion, DEAE dextran, calcium phosphate precipitation, or use of a gene gun (see for instance Wu et al., 1992 ; Wu et al, 1988).

The donor nucleic acid targeting system can also be introduced by lipofection. In certain embodiments, the use of liposomes and/or nanoparticles is contemplated for the introduction of the donor nucleic acid targeting system into host cells. Nanocapsules can generally entrap compounds in a stable and reproducible way. Ultrafine particles (sized around 0.1 µm) that can be designed using biodegradable polyalkyl-cyanoacrylate polymers are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The use of cationic lipids may promote encapsulation of negatively charged nucleic acids, and also promote fusion with negatively charged cell membranes (Felgner et al., 1989).

*In vivo* targeted gene delivery is described in international patent publication WO 95/28 494. Alternatively, the vector can be introduced *in vivo* by lipofection, using liposomes or nanoparticles as above described. It is also possible to introduce the vector *in vivo* using techniques that are similar to the techniques that are employed *in vitro (e.g.* transfection, electroporation...).

### Transformed cells

The invention further relates to a transformed host cell that contains:
- a first nucleic acid sequence comprising a cDNA encoding a retrovirus core protein ;
- a second nucleic acid sequence comprising a cDNA encoding a F protein of hMPV;
- possibly a third nucleic acid sequence comprising a packaging competent retrovirus-derived genome.

In an embodiment, the second nucleic acid sequence comprises a cDNA encoding a F protein and a G protein of hMPV. As an alternative, two second nucleic acid sequences are present one comprising a cDNA encoding a F protein the other comprising a cDNA encoding a G protein.

The core proteins from said retrovirus may be encoded either by the *gag* and *pol* genes or by the *gag* gene alone.

Such a transformed host cell is obtainable as described in a method above.

In another aspect, the invention relates to the use of a transformed host cell as defined above, for the identification of molecules capable of interfering with hMPV entry in cells. The invention provides in particular a method of *ex vivo* screening or identification of molecules capable of interfering with hMPV entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule, under conditions that allow syncytia formation, *i.e.* cell-cell fusion, and infectious chimeric virus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with hMPV entry the candidate molecule for which syncytia formation, as measured in the presence of said molecule, is decreased as compared to syncytia formation measured in the absence of any candidate molecule.

Contacting a transformed host cell with a target host cell, and a candidate molecule can be carried out by contacting simultaneously said transformed host cell, target host cell and candidate molecule. Otherwise, two of these three elements can be contacted under conditions sufficient to allow their interaction before addition of the third missing element.

Preferably said target host cell is not transformed, *i.e.* said target host cell does not contains at least one of the first, second, and third nucleic acid sequence as defined above.

Syncytia formation can be readily assessed by one skilled in the art. Briefly, the coculture is submitted to an acidic pH drop by incubation for 5 min at pH-5 and incubated in a normal medium for an additional 12 hrs. Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

### Chimeric virus-like particles

In the method described above no structural modifications of the F and/or G hMPV glycoproteins are required for their correct assembly on retroviral cores. The method of the invention thus makes it possible to generate high titer pseudo-particles with functional F and/or G glycoproteins. As demonstrated herein, the infectious particles exposing a F protein constitute a valid model of hMPV virion as regards to early steps of viral infection cycle.

The invention further relates to a chimeric virus-like particle. Such a particle is obtainable *ex vivo* or *in vivo* by a method as described above.

An object of the invention is thus a chimeric virus-like particle, comprising a retrovirus core protein, and a human metapneumovirus (hMPV) F and/or G protein.

In an embodiment, this chimeric virus-like particle comprises a hMPV F protein. This particle may comprise the F protein as sole hMPV protein. The F protein is preferably unmodified. It is functionally incorporated on the particles. The F protein includes the transmembrane domain. The F protein preferably comprises the whole, unmodified extracellular domain. As explained before, the presence of a fragment or part thereof is also within the scope of this invention.

In another embodiment, this chimeric virus-like particle comprises both hMPV F and G proteins.

In another embodiment, a chimeric virus-like particle comprising hMPV G protein is provided.

In a preferred embodiment, this chimeric virus-like comprises the F protein under the F0 form or under the form of the F1 and F2 subunits (ref. 49).

In the invention, the hMPV proteins may correspond to proteins from one or several hMPV sub-groups. Thus, the chimeric virus-like particle may comprises F protein from sub-group A hMPV, from sub-group B hMPV or from both sub-group A hMPV and sub-group B hMPV. Thus, the chimeric virus-like particle may comprises F and G protein from sub-group A hMPV, from sub-group B hMPV or from both sub-group A hMPV and sub-group B hMPV.

According to different embodiments, the chimeric virus-like particle comprises a core protein of MLV, ALV, RSV, MPMV, HIV-1, HIV-2, SIV, EIAV, CAEV, or HFV. In an embodiment, it is MLV.

The core protein may be Gag and/or Pol. In an embodiment, the retrovirus core protein is gag. In another embodiment, the core protein comprises Gag and Pol.

In an embodiment, the VLP comprises Gag and Pol and hMPV F. This VLP is advantageously infectious.

In a specific embodiment, the infectious chimeric virus-like particle further comprises a packaging competent retroviral-derived genome. In this embodiment, preferably, the Gag and Pol proteins are present. Preferably, this packaging competent retroviral-derived genome further comprises a transgene.

Advantageously, said particles further carry a transgene. For instance said transgene may be a marker gene which make it possible to follow-up cell infection by the infectious particles of the invention and can find application for instance in the identification of a cell receptor involved in hMPV entry. Said transgene can also be a gene encoding a molecule of therapeutic interest and/or a suicide gene. Accordingly, the particles of the invention that specifically target mammalian mononuclear macrophages, monocytes or dendritic cells, endothelial cells, fibroblasts, neurone/neuroblastoma cells, hepatocyte/hepatoma cells comprise a useful vector for gene transfer and/or gene therapy.

### Uses of the infectious chimeric virus-like particles of the invention

High infectivity of these particles makes it possible for the investigation of the role of hMPV F and G glycoproteins and their potential receptors in cell entry, hMPV host-range and neutralisation by antibodies from hMPV patient sera.

The invention therefore concerns the use of a virus-like infectious particle as described above, for *ex vivo* identification of a cell receptor for hMPV F and/or G glycoprotein.

According to an embodiment, the invention provides a method for *ex vivo* identification of a receptor for hMPV F and/or G glycoprotein comprising detection of the binding of said particle to a cell receptor. More specifically, the method may comprise the steps consisting of:
- contacting a cell susceptible to hMPV infection with an infectious virus-like particle of the invention, under conditions sufficient to allow specific binding of said particle to a receptor expressed at the surface of said cell;
- detecting binding of said particle to a receptor; and
- identifying said receptor.

In an embodiment, the method is applied to F alone.

A cell susceptible to hMPV infection may preferably be selected from the group consisting of mammalian mononuclear macrophages, monocytes or dendritic cells, endothelial cells, fibroblasts, neurone/neuroblastoma cells, hepatocyte/hepatoma cells.

Detection of particle binding to a receptor can be achieved according to classical procedures well-known by one skilled in the art. For instance, this could involve radioactive, enzyme or fluorescent labeling of the particles of the invention, and subsequent detection with an appropriate method. A number of fluorescent materials are known and can be utilized as labels. These include, for example, fluorescein, rhodamine, auramine, Texas Red. Enzyme labels consist in conjugation of an enzyme to a molecule of interest, e.g. a polypeptide, and can be detected by any of colorimetric, spectrophotometric, or fluorospectrophotometric techniques. Flow cytometry analysis (FACS) together with labeled antibodies directed against F and/or G proteins harboured by the pseudo-particles of the invention is also appropriate.

According to another embodiment, the invention provides a method for *ex vivo* identifying a cell receptor for hMPV comprising the step consisting of:
- transfecting a cell which is not permissive for hMPV infection with a nucleic acid sequence encoding a protein likely to be a hMPV receptor;
- contacting said transformed cell with a hMPV-like particle of the invention;
- determining whether said transformed cell has become permissive or not for hMPV infection; and
- identifying as a cell receptor for hMPV said protein expressed by the transformed cell that has become permissive.

Determination of whether the transformed cell has become permissive for hMPV infection can be readily achieved using the virus-like particles of the invention. In particular, where said particles carry a marker gene, such as GFP, permissivity (*i.e.* the capacity of cells to be infected with hMPV, and in particular with the virus-like particles) can be assessed by FACS analysis of the transformed cells. Where the marker gene is an antibiotic resistance gene, identification of cells infected by hMPV-like particle is readily achieved through exposure to said antibiotic.

Where one does not suspect a given protein to be a receptor for hMPV entry in cells, the above method can advantageously be adapted for the screening and the identification of a cell receptor for hMPV. In particular, an expression cDNA library can be prepared, for instance from a cDNA library obtained by reverse-transcription of cellular mRNAs from a cell permissive for hMPV infection. Expression of such a cDNA library would be driven by a constitutive promoter whose nucleic acid sequence has been fused to the cDNA library in suitable vectors. Such a library would contain a vector encoding a cell receptor for hMPV. Non permissive cells can then be transfected with this expression library and further screened for the identification of a cell receptor for hMPV.

To this end, the invention proposes a method for *ex vivo* identifying a cell receptor for hMPV comprising the step consisting of:
- providing an expression cDNA library obtained from a cell permissive for hMPV infection;
- transfecting cells that are not permissive for hMPV infection with said expression cDNA library;
- contacting said transformed cells with virus-particles of the invention;
- identifying and isolating those transformed cells that have become permissive for hMPV infection;
- isolating the expression vector transfected in cells that have become permissive; and
- identifying as a receptor for hMPV the proteins encoded by the cDNA sequence of said isolated expression vectors.

Determination of whether the transformed cell has become permissive for hMPV infection can be readily achieved using the virus particles of the invention. In particular, where said particles carry a marker gene, such as GFP, permissivity (*i.e.* the capacity of cells to be infected with hMPV, and in particular with virus-like particles) can be assessed by FACS analysis of the transformed cells. Where the marker gene is an antibiotic resistance gene, identification of cells infected by virus-like particles is readily achieved through exposure to said antibiotic.

Advantageously, the expression cDNA library is expressed from retroviral vectors that comprise glycoproteins that allow infection of the hMPV non permissive cells. Such glycoproteins can be the VSV-G glycoprotein derived from vesicular stomatitis virus (VSV) whose receptor is expressed in most cell types *ex vivo.* Such viral particles can be assembled using a packaging competent retrovirus-derived genome that comprises the expression cDNA library, and optionally a marker gene. According to this embodiment the method for isolating the expression vector expressed in cells that have become permissive to infection by the virus-like particles of the invention is greatly facilitated. Indeed this latter embodiment is particularly advantageous in that the process of cell infection with retroviral vectors has greater efficacy, as compared to cell transfection. Furthermore, cell infection leads to stable integration of viral genome in the cellular genome. Accordingly, transgenes, *i.e.* cDNA, and marker gene that are carried by the pseudo-particles of the invention, are found to be stably expressed by infected cells. This is in contrast with classical vectors used for transfection that do not integrate into cellular genome and for which expression may be transient.

In another aspect, the invention relates to the use of an infectious particle as defined above, for the identification of molecules capable of interfering with hMPV entry in cells. In particular, herein is provided a method of *ex vivo* screening or identification of molecules capable of interfering with hMPV entry in cells comprising comparison of the level of cell infection by the virus-like particles of the invention in the presence or the absence of a candidate molecule. Said method preferably comprises the steps consisting of:
- contacting a cell susceptible to hMPV infection with an infectious virus-like particle, in the absence or presence of a candidate molecule, under conditions that allow cell infection with virus-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with hMPV entry the candidate molecule for which cell infectivity, as measured in the presence of said molecule, is decreased as compared to cell infectivity measured in the absence of any candidate molecule.

Contacting a cell susceptible to hMPV infection with an infectious virus-like particle, and a candidate molecule can be carried out by contacting simultaneously said cell, virus-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious virus-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with hMPV cell entry may be any permissive cell for hMPV infection, as described above.

Such molecules capable of interfering with hMPV entry in cells may constitute new antiviral drugs.

### Diagnosis

The VLPs of the invention are further useful for diagnosis of hMPV infection and follow-up of hMPV infection, for instance to assess efficacy of a therapy in a patient.

The invention thus concerns the use of a virus-like particle for the *in vitro* detection of antibodies directed against hMPV in a biological sample from a subject susceptible to be infected with hMPV. Said biological sample may be a biological fluid, such as blood or serum, or a tissue biopsy. In a specific embodiment, said antibodies are directed against F and/or G hMPV glycoproteins. In an embodiment, said antibodies are directed against F.

Accordingly, the invention provides a method of *in vitro* diagnosis of an hMPV infection in a patient comprising detecting immune complexes formed by interaction of anti-hMPV antibodies likely to be present in a biological sample of the patient, with virus-like particles of the invention. Said method may in particular comprise the steps consisting of:
- contacting a biological sample with a virus-like particle of the invention under conditions sufficient to allow formation of complexes by binding of said particle to antibodies directed against hMPV present in the biological sample;
- detecting said complexes, which presence is indicative of an hMPV infection.

The presence of antibodies reactive with virus-like particles can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the virus-like particle and the antibody or antibodies reacted therewith.

In another embodiment, said method of *in vitro* diagnosis of an hMPV infection in a patient comprises detecting an inhibitory effect of anti-hMPV antibodies likely to be present in a biological sample of the patient, on the infection of a permissive cell by a virus-like particle of the invention. Said method may in particular comprise the steps consisting of:
- contacting a cell permissive for hMPV infection with a virus-like particle and a biological sample;
- comparing cell infectivity measured in presence of said biological sample with cell infectivity measured in absence of said biological sample;
- detecting the inhibition of virus-like particle infection of a permissive cell as a decrease in cell infectivity measured in presence of said biological sample compared with cell infectivity measured in absence of said biological sample, said inhibition being indicative of an hMPV infection.

This embodiment is advantageous in that the method relies on the detection of the specific antibodies that are neutralizing for cell infection, that is those patient's antibodies that are effective against viraemia.

In a further embodiment of this invention, commercial diagnostic kits may be useful to carry out the above diagnosis methods, by detecting the presence or absence of immune complexes formed by hMPV particles and antibodies directed against hMPV in a biological sample from a subject susceptible to be infected with hMPV, or by detecting an inhibition of virus-like particle infection of a permissive cell by anti-hMPV neutralizing antibodies likely to be present in a biological sample of the patient. Such kits may comprise at least a virus-like particle of the present invention. Where the method involves detection of immune complexes, the kits may further comprise appropriate means of detection of said immune complexes. Preferably the kit of the invention further comprises directions, and protocols, depending upon the method selected, e.g., "competitive", "sandwich", and the like. The kits may also contain peripheral reagents such as buffers, stabilizers, etc.

### Immunogenic compositions and vaccines

In another aspect of the invention, the chimeric virus-like particles of the invention may be used for immunization or vaccination purposes.

An object of the invention is thus an immunogenic or vaccine composition comprising:
- a chimeric virus-like particle as described herein and a pharmaceutically acceptable carrier or vehicle, or
- an expression vector or expression vectors in a pharmaceutically acceptable carrier or vehicle able to produce *in vivo* a chimeric virus-like particle as described herein.

According to an embodiment, the invention thus proposes also a method of immunization, notably against hMPV infection, that comprises administration of such an immunogenic composition to a subject in need thereof. The method comprises the administration to a subject in need thereof of a sufficient amount of said composition.

According to an embodiment, the invention thus proposes also a method of vaccination, notably against hMPV infection, that comprises administration of such a vaccine composition to a subject in need thereof. The method comprises the administration to a subject in need thereof of a sufficient amount of said composition.

In an embodiment, the chimeric virus-like particle comprises hMPV F protein. This particle may comprise the F protein as sole hMPV protein.

In another embodiment, this chimeric virus-like particle comprises both hMPV F and G proteins.

In another embodiment, the composition comprises a chimeric virus-like particle comprising hMPV F protein and a chimeric virus-like particle comprising hMPV G protein.

In a preferred embodiment, this chimeric virus-like comprises the F protein under the F0 form or under the form of the F1 and F2 subunits.

In the invention, the hMPV proteins may correspond to proteins from one or several hMPV sub-groups. Thus, the chimeric virus-like particle may comprises F protein from sub-group A hMPV, from sub-group B hMPV or from both sub-group A hMPV and sub-group B hMPV. Thus, the chimeric virus-like particle may comprises F and G protein from sub-group A hMPV, from sub-group B hMPV or from both sub-group A hMPV and sub-group B hMPV.

According to different embodiments, the chimeric virus-like particle comprises a core protein of MLV, ALV, RSV, MPMV, HIV-1, HIV-2, SIV, EIAV, CAEV, or HFV. In an embodiment, it is MLV.

The core protein may be Gag and/or Pol. In an embodiment, the retrovirus core protein is gag. In another embodiment, the core protein comprises Gag and Pol.

The vaccine and immunogenic compositions of the invention may be drawn to confer immunity, or elicit an immune response against hMPV.

However, where the virus-like particles of the invention further carry an additional gene encoding another antigen, different from hMPV antigens, the invention provides a recombinant viral vaccine useful to raise an immune response against said antigen. Actually, the use of pseudo-particles described herein makes it possible to improve the elicited immune response through combining several presentations and processing pathways of an antigen. For instance, a vaccine composition of the invention, when administered, results in the virus-like particles infecting cells of the host. The transgene encoding the antigen is then integrated in the cellular genome, and subsequently expressed by the cell, such that there is both a cellular and a humoral immune response elicited by the vaccine composition.

Advantageously, the virus-like particles may further carry a transgene encoding an immune modulator, which allows for enhancement of the raised immune reaction.

The vaccination or immunogenic composition of the present invention may additionally contain an adjuvant. A number of adjuvants are known to those skilled in the art. Examples of suitable adjuvants include, for example, aluminum hydroxide; Saponin; detergents such as Tween 80; animal, mineral or vegetable oils, Corynebacterium or Propionibacterium-derived adjuvants; Mycobacterium bovis (Bacillus Calmette and Guerin, or BCG); cytokines; acrylic acid polymers such as carbomer (Carbopol®); EMA; or combinations thereof.

Subjects in need thereof may be in particular young children, for example children less than 1 year, or less than 6 months of age, immuno-compromised individuals and the elderly. Vaccination of young children may be made shortly after birth, for example between 1 and 6 months, e.g. at 2 months. Young children may be vaccinated at a stage at which they have maternal antibodies.

The vaccination may comprise one or several administrations, preferably at least two at a given interval, typically two.

The route of administration is any conventional route used in the vaccine field. As general guidance, a vaccine composition of the invention is administered via a mucosal surface, e.g., an ocular, intranasal, pulmonary, oral, intestinal, rectal, vaginal, and urinary tract surface; or via a parenteral route, e.g., by an intravenous, subcutaneous, intraperitoneal, intradermal, intraepidermal, or intramuscular route. The choice of administration route depends on the formulation that is selected.

The invention will be further understood in view of the following examples and the annexed figures.

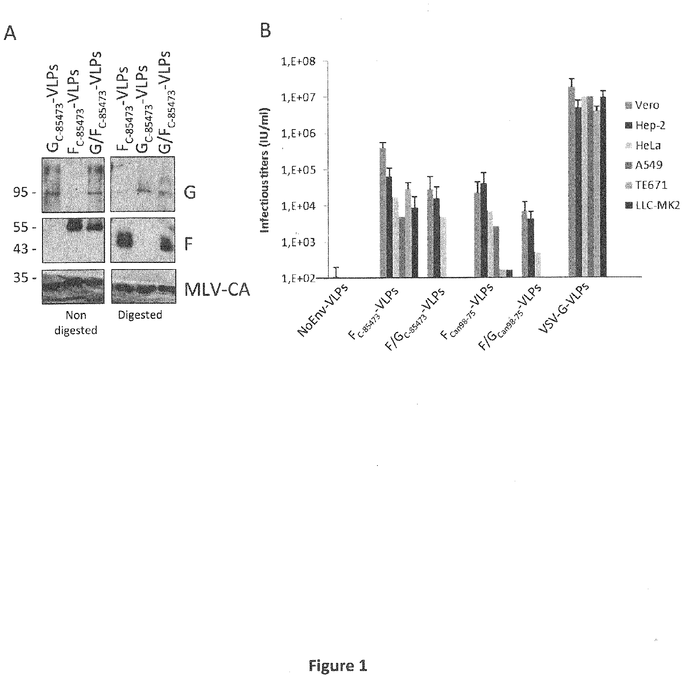

**Figure 1****.** Production and characterization of human metapneumovirus virus-like particles (hMPV-VLPs).
   A. Biochemical analysis of hMPV-VLPs generated by individual or co-expression of hMPV F and G glycoprotein, as indicated, and purified by ultracentrifugation over a sucrose cushion. Upon separation by SDS-PAGE, the incorporation levels of F and G on MLV core particles were determined by Western blot analysis using a polyclonal serum against hMPV. hMPV-VLPs were analyzed before and after trypsin digestion, as indicated.
   B. Infectious titers on various cell lines obtained with hMPV-VLPs incorporating surface proteins from C-85473 or Can98-75 hMPV or with VSV-G-VLPs incorporating the G glycoprotein from vesicular stomatitis virus (VSV). The transduction efficiency, determined as the percentage of GFP-positive cells, was measured by FACS analysis and is provided in GFP-infectious units (IU) per milliliter (mean ± SD; n=4).

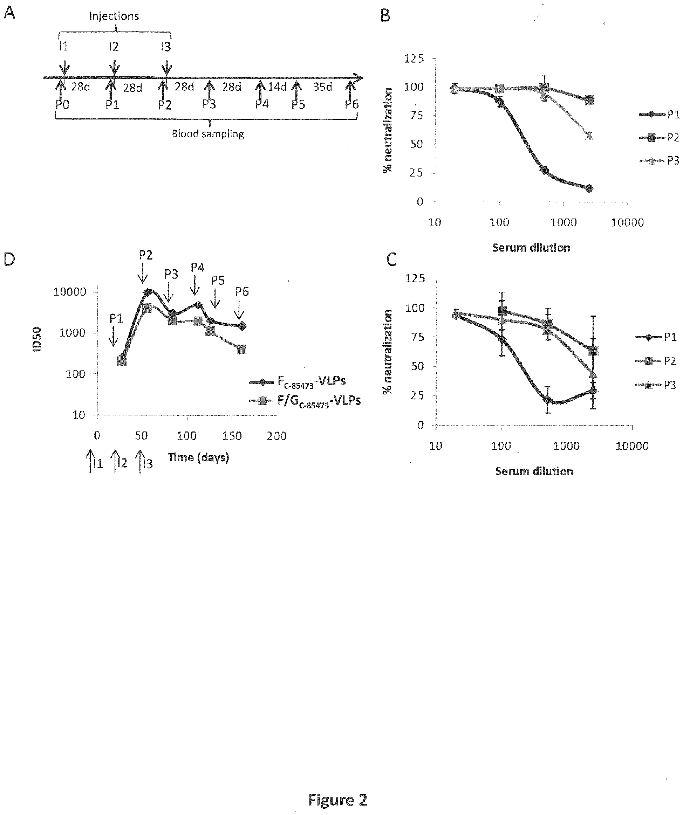

**Figure 2****.** Humoral immune responses induced by hMPV-VLPs.
   A. Outline of the immunization protocol. Balb/c mice were inoculated 3 times (11 to 13), at 28-days intervals, by IP injection of hMPV-VLPs displaying F, G, or F/G hMPV glycoproteins from C-85473 hMPV strain. At different time points following immunization, blood samples were harvested (P1 to P6) to determine the production of neutralizing antibodies.
   B and C. The neutralization curves of sera from mice immunized with F-VLPs (B) and F/G-VLPs (C), harvested 28 days after each injection (P1, P2 and P3), were determined for different serum dilutions (1/20, 1/100, 1/500 and 1/2,500). The results are expressed as the neutralization levels (mean ± SD; n = 4) determined in the immune sera relative to P0 pre-immune sera.
   D. Doses of 50% inhibition (ID50) obtained via immunization with F-VLPs and F/G-VLPs were deduced from the neutralization curves that were determined until P6, i.e. 161 days after the first immunization (A).

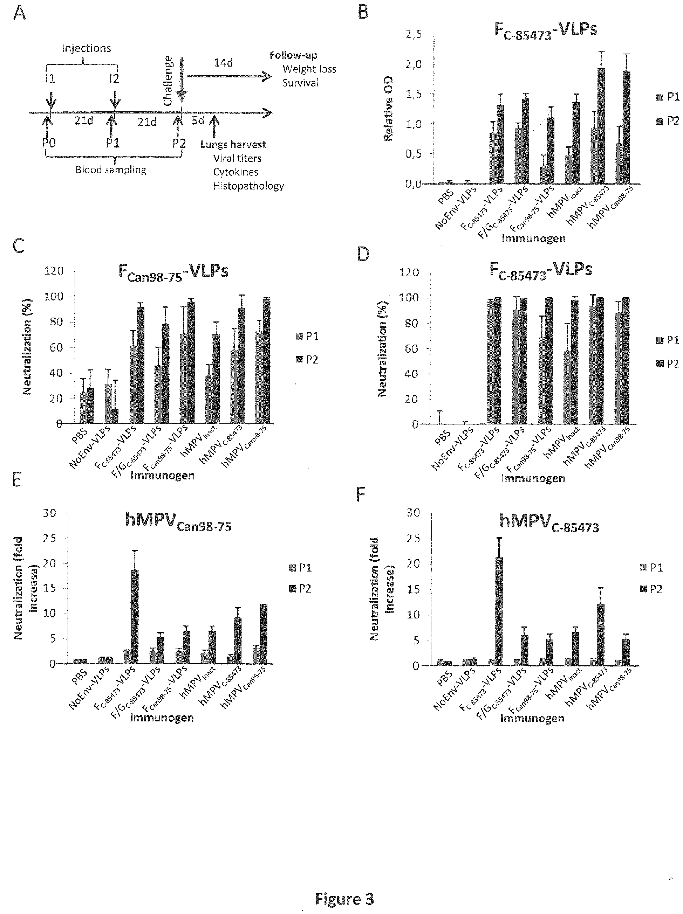

**Figure 3****.** Induction of antibodies in the serum of VLP-vaccinated mice.
   A. Outline of the immunization protocol. Balb/c mice were vaccinated 2 times (I1 and I2), at a 21-days interval, with hMPV-VLPs displaying F, G, or F/G hMPV glycoproteins from either C-85473 or Can98-75 strains. Control mice were vaccinated with PBS only, with VLP devoid of glycoproteins (VLP no Env), with hMPV inactivated with formaline (hMPV inact) or with live hMPV from C-85473 and Can98-75 strains. 21 days after each inoculation, blood samples were harvested (P1 and P2) to determine the production of antibodies. 21 days after the last immunization, mice were intranasally infected with C-85473 and Can98-75 hMPV (challenge).
   B. An hMPV-F_{C-85473} ELISA was performed on the blood samples to detect F antibodies using, as antigen, F_{C-85473}-VLP coated in wells.
   C and D. The neutralization efficiency was determined for 1/100 serum of blood samples, using murine leukemia virus (MLV) vector particles incorporating F_{Can98-75}-VLPs (C) or F_{C-85473}-VLPs (D). Results are expressed as percentages of neutralization (mean standard deviation; n=6) of the transduction titers relative to P0 pre-immune serum samples.
   E and F. Neutralizing antibodies present in P1 and P2 serum samples were evaluated by plaque reduction virus neutralization titers against Can98-75 (E) and C-85473 (F). Results are expressed as mean reciprocal log² titers±SD relative to titers obtain with PBS.

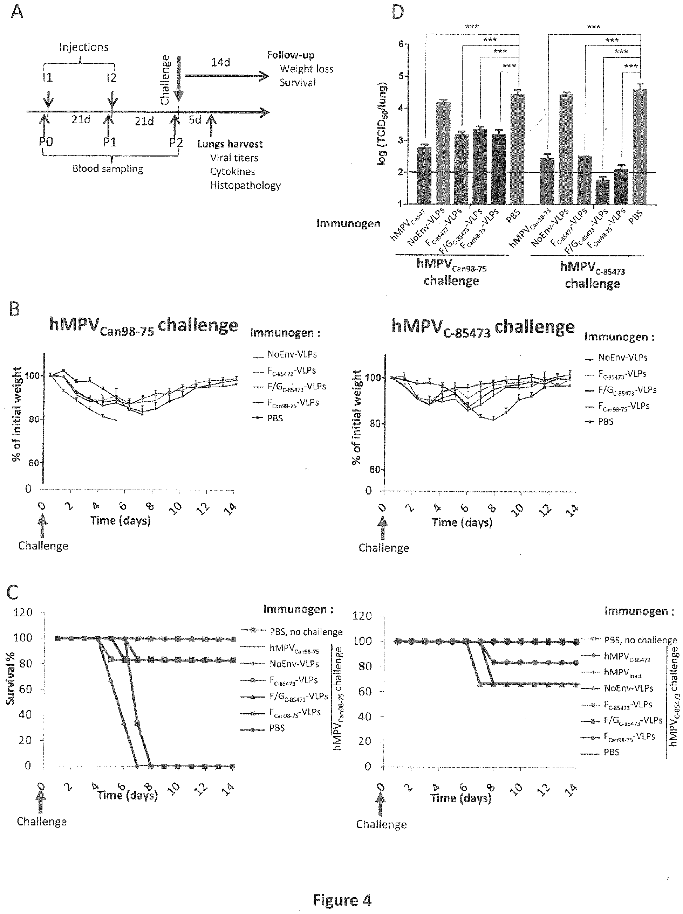

**Figure 4****.** Protection of hMPV-VLP-vaccinated mice against hMPV challenge.
   A. Outline of the immunization protocol as in figure 3A.
   B and C. Average weight loss (B) and survival rates (C) of immunized mice after challenge. The results were obtained from 6 mice per group and were monitored fro 14 days after challenge with hMPV Can98-75 (left panels) or C-85473 (right panels). Naive mice, inoculated with PBS and not challenged, were included as a control group.
   D. Infectious virus titers in the lungs of mice challenged with hMPV Can98-75 or C-85473, as indicated. Lungs were collected 5 days post-infection and virus present in tissues was quantified by serial dilutions on LLC-MK2 cells. Virus titers were reported as log₁₀ TCID₅₀ (g lung)⁻¹.

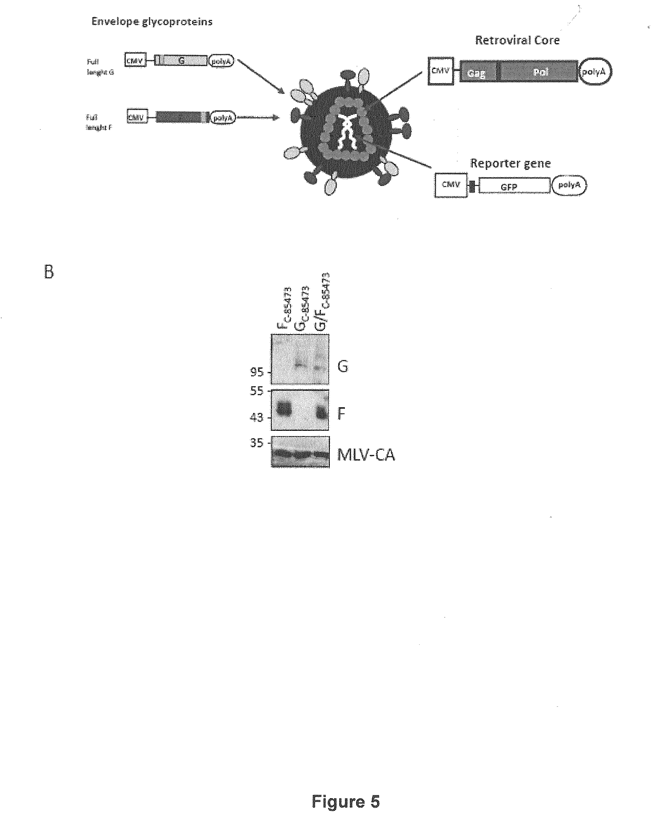

**Figure 5****.** Production of infectious chimeric virus-like particles or hMPV-VLP. Schematic representation of hMPV-VLP and DNA constructs that upon transfection allow to induce hMPV-VLPs production. CMV, cytomegalovirus promoter; GagPol, Friend-murine leukemia virus (MLV) GagPol proteins, respectively; F and G, fusion and haemagglutinin of hMPV Can9875 or C-85473.

### Materiels and methods

**Cell and viruses.** 293T cells, Vero cells, Hep2 cells, LLC-MK2 cells, A549 cells and TE671 were grown in Dulbecco's modified Eagle medium supplemented with 10% fetal calf serum and 1% penicillin/streptomycin. hMPV C-85473 are Can98-75 are clinical strains passaged seven times in LLC-MK2 cells using Opti-MEM medium (Gibco) supplemented with 0.0002% trypsin (Sigma) and 0.2% gentamicin (Gibco) (hMPV infection medium).

**Expression constructs.** The expression vectors pTG5349 (Friend-murine leukemia virus [MLV] GagPol) and pTG13077 (MLV-based transfer vector coding for green fluorescent protein [GFP]) were described elsewhere (20, 36, 39). Sequences encoding F_{C-85473}, F_{Can98-75}, G_{C-85473} and G_{Can98-75} were amplified by PCR using 5'-attcggccgCTCGAGcaccatggcttcttggaaagtggtgatcattttttcattgttaata-3' (SEQ ID NO:1) and a 5'-attcggccgAGATCTctaattatgtggtatgaagccattgtttgtgac-3'(SEQ ID NO:2), 5'attcggccgCTCGAGcaccatggcttcttggaaag-tgatgattatcatttcgttactcata-3' (SEQ ID NO:3) and 5'-attcggccgAGATCTctaactatgcggtataaaaccgccgttggtaac-3' (SEQ ID NO:4), 5'-attcggccgCTCGAGcaccatggag-gtgaaagtggagaacattcgaacaataga-3' (SEQ ID NO:5) and 5'-attcggccgAGATCT-ttaactagtttggttgtatgttgttgatgtgctg-3' (SEQ ID NO:6), 5'-attcggccgCTCGAGcaccatggaagcaagagtggagaacattcgggcaataga-3' (SEQ ID NO:7) and 5'-attcggccgAGATCTttaactacttggagaagatgtgtctgtgttgtgt-3' (SEQ ID NO:8) primers respectively and cloned in BamHI/Xhol restriction sites in phCMV vector.

**Virus-Like Particle production and infection.** 293T cells were transfected with expression vectors encoding viral GPs, retroviral core proteins, and GFP transfer vector using calcium phosphate. Twenty-four hours after transfection, the medium was replaced with Dulbecco's modified Eagle's medium-1 0% fetal calf serum. Supernatants were harvested 24 h after changing the medium and filtered (0.45-µm pore size). Before infection of target cells, VLPs were digested 5 min with 0,7 µg/ml of TPCK trypsin, trypsin was inactivated with 1% FCS. Serial dilutions of viral supernatants were added to the cells and incubated for 7 h. Supernatants were then removed, and the infected cells were kept for 72 h before analysis of the percentage of GFP-positive cells by fluorescence-activated cell sorter (FACS) analysis.

**Western blot.** Cell supernatant was harvested 48 h after transfection. hMPV-VLPs were concentrated on a 20% sucrose cushion by ultracentrifugation (110,000 *g* for 2 h; Beckman SW41) to 1/100 of the initial volume, were separated by sodium dodecyl sulfate-polyacrylamide gel electrophoresis, and were transferred onto nitrocellulose membranes (Optitran BA-S 85; Schleicher & Schuell). Antibodies against hMPV (mice antiserum against the hMPV C-95473) and against MLV Gag (rat antiserum against the MLV capsid protein) were used to detect hMPV envelope glycoproteins and MLV capsid. HRP-conjugated anti-mouse and anti-rat were used as secondary antibodies.

**Vaccination and hMPV challenge.** Groups of 18 4-6-week-old BALB/c mice (Charles River Laboratories) were immunized 2 times by intraperitoneal injection, at a 21 days interval, with 100µL of 100 fold concentrated hMPV-VLPs incorporating F_{C-85473} , F/G_{C-85473}, F_{Can98-75} or no envelope. Control mice were vaccinated with 1x10⁶ TCID 50 of hMPV C-85473, 1x10⁶ TCID 50 of hMPV Can98-75, 1x10⁶ TCID 50 hMPV inactivated with formalin (hMPV inact) and PBS. 21 days after each vaccination a blood sample was taken to evaluate the production of antibodies (P1 and P2). 21 days after the last immunization, mice were intranasally infected with 1x10⁸ TCID50 hMPV strains C-85473 or Can98-75 as described previously (18). Daily, 6 mice per group were weighed and a follow up was performed to detect signs of disease for 14 days after challenge. On day 5 post-infection, lungs were removed from another 6 mice per group and viral titers in the lungs were determined as well as cytokine levels. Lung samples from the last 6 mice per group were collected for histopathological studies.

**VLP microneutralization assay.** Serum samples from vaccinated mice were heat inactivated at 55°C for 1 h, and neutralizing antibody titers were determined. Briefly, infectious hMPV-VLPs harboring an MLV-based transfer vector coding for GFP were produced, incubated with diluted serum samples from vaccinated mice, and mixed with Vero target cells in a 96-well plate. The transduction efficiency was determined as the percentage of GFP-positive cells by high-throughput fluorescence-activated cell sorter analysis 72 h after transduction. The presence of neutralizing antibodies was determined as the reduction of infectious titers induced by P1 or P2 immune serum samples relative to P0 preimmune serum samples.

**hMPV neutralizing-antibody assay.** hMPV neutralizing-antibody titres were determined by an end-point dilution assay (37). Briefly, blood samples were centrifuged (1200 r.p.m., 10 min), then sera were collected and incubated at 56°C for 30 min to inactivate non-specific inhibitors. Twofold dilutions of sera were performed in quadruplicate in hMPV infection medium containing fresh trypsin, and then mixed with an equal volume of infection medium containing 500 TCID50 hMPV strain C-85473 or Can98-75. The antibody-virus mixtures were incubated at 37°C for 2 h and then transferred into 24-well plates containing LLC-MK2 cells. Following incubation for 5 h, the mixtures were removed and 500 µl fresh infection medium was added to each well. Plates were incubated at 37°C and, 3 days later, another 500 µl infection medium was added with fresh trypsin. Neutralization titers were defined as the highest dilution of antibody at which culture wells were negative for infection. hMPV neutralizing-antibody titers were reported as mean reciprocal log² titres±SD. The lower limit of detection of this assay is 3 log².

**ELISA.** Antigens used as targets in ELISA were F_{C-85473}-VLPs and controls were VLPs harboring a heterologous MLV-A envelope. Microtiter plates were coated with lectin from *Galanthus nivalis* (Sigma) in carbonate buffer over night at 4°C. After washing, antigen VLPs were diluted (1/2) in TBS, added to micro titer wells and incubated overnight at 4°C. After binding of the target antigens, wells were washed and blocked in TBS-2,5% BSA for 1 hour at room temperature. Serum sample were diluted in TBS-tween-2,5% BSA, added to the wells and incubated for 2 hours at room temperature. After washing of the wells, a HRP-conjugated anti-mouse antibody (Southern Biotech) was added and incubated for 1 hour at room temperature. After washing, TMB substrate was added, reaction was stopped with 1 N H₂SO₄ and the 450 nm optical density was read in a plate reader

**Virus titers in lungs.** On day 5 post-infection, which is the time of maximal viral replication in lungs of mice (18), animals were euthanized and their lungs were removed and frozen quickly in liquid nitrogen. Subsequently, lungs were weighed and homogenized in hMPV infection medium, and viral titres were determined by performing 10-fold serial dilutions of lung homogenates in 24-well plates containing LLC-MK2 cells. Before infection, cells were washed twice with PBS. Infected plates were incubated at 37°C with 5% CO2 and replenished with 1 ml fresh trypsin (0.0002 %) every other day. Virus titres were reported as log₁₀ TCID₅₀ (g of lung)⁻¹. The lower limit of detection of this assay is 10² TCID₅₀ (g lung)⁻¹. TCID50 values were calculated by the standard method of Reed & Muench (1938).

### Results

Incorporation of F and G glycoproteins onto retroviral VLPs leads to infectious particles. Since heterologous glycoproteins are not necessarily efficiently incorporated on retroviral cores, we first evaluated whether hMPV glycoproteins could be displayed on retrovirus-derived virus-like particles (VLP). To generate such hMPV-VLPs, 293T cells were co-transfected with four expression vectors encoding the murine-leukemia virus (MLV) Gag-Pol proteins, a packaging-competent MLV-derived genome encoding the green fluorescent protein (GFP) and the F envelope glycoprotein and/or G envelope glycoprotein from the C-85473 (belonging to the A linage) strain of hMPV. VLPs were harvested from the supernatant of transfected cells and were digested with trypsin to be processed into F1 and F2 subunit. Digested and native viral particles were then purified and concentrated by ultracentrifugation through high-density sucrose cushions and characterized by immunoblot using a mouse serum raised by infection with hMPV strain C-85473. The results showed incorporation of F protein and G of protein (Figure 1A). G protein was detected at a molecular weight of about 100 kDa for both digested or not digested VLPs. The uncleaved F0 form was detected at the expected molecular weight of 60 kDa, while the F1 form was detected at a molecular weight of 47 kDa for the trypsin-digested VLPs (Figure 1A). We noticed that incorporation of F was slightly lower when G was co-expressed and co-incorporated. An identical strategy was used for F and G from hMPV strain Can98-75 (B lineage); yet, the detection of either glycoprotein with anti the C-85473 serum was not possible, suggesting a low cross-reactivity of this serum.

The tropism of hMPV remains poorly clarified. Thus, in order to identify appropriate target cells and to demonstrate that F and G incorporated on VLPS were functional, hMPV-VLPs were titrated onto various cell types (Figure 1 B). Since the hMPV-VLP were generated with replication-defective viral components encoding a GFP marker, their infectivity could be precisely determined by FACS analysis. After 7 h of incubation with target cells, the viral supernatants were replaced with normal medium, and the infected cells were analyzed for GFP expression 72 h later. We found that hMPV-VLPs harboring F and G proteins (F/G-VLPs) or F alone (F-VLPs) from either C-85473 or Can98-75 strains were functional and mediated entry into cells (Figure 1 B). In contrast, VLPs displaying hMPV-G proteins alone did not result in infectious particles (data not shown). Higher titers (to up to 5x10⁵ IU/ml) were detected on Vero and Hep2 cells as compared to other cell types. Moreover, hMPV-VLPs harboring the glycoproteins from C-85473 strain were more infectious than those incorporating the glycoproteins from Can98-75 strain. Surprisingly, although the G protein is known as an attachment protein (42), it was clearly dispensable and higher infectious titers were obtained for F-VLPs as compared to F/G-VLPs. On HeLa, TE671, A549, LLC-MK2 and 293T, the titers of F/G-VLPs and F_{Can98-75}-VLPs were too low (<10² IU/ml) to be detected.

Altogether, these results indicated that F and G can be functionally displayed on the surface of VLPs and that F is a necessary and sufficient protein that mediates cell entry.

hMPV-VLPs induce humoral immune responses in mice. We then investigated whether hMPV-VLPs displaying viral proteins could induce specific neutralizing antibody responses against hMPV in balb/c mice. F_{C-85473}-VLPs and F/G _{C-85473}-VLPs were concentrated and purified by ultracentrifugation over a sucrose cushion, and were digested with trypsin before intraperitoneal injection. Control VLPs, carrying no hMPV glycoproteins (NoEnv-VLPs) were also prepared and inoculated in mice in parallel. As described in figure 2A, mice were immunized 3 times at 28-day intervals (figure 2A). Serum samples were collected before the first immunization (P0) and 28 days after each injection (P1, P2 and P3). In order to determine the neutralizing activity of the sera, we titrated the infectivity of hMPV-VLPs harboring a GFP marker gene after pre-incubation with mice sera and subsequent infection of Vero target cells. The results of a typical experiment, displayed as the % of neutralization of dilutions of the sera collected at P1, P2 or P3 relative to the P0 pre-immune sera, are shown in figure 2B (inoculation of F-VLPs) and 2C (inoculation of F/G-VLPs). We found that P1 sera, harvested 4 weeks after the first injection, had significant neutralizing activity, with more than 75% neutralizing activity obtained at a 1/100 dilution. The P2 and P3 sera had much higher neutralizing activity, allowing more than 95% neutralization even at a 1/500 dilution. Of note, the third injection did not increase the neutralizing activity of the sera. The neutralization curves obtained for sera of mice inoculated with F/G-VLPs exhibited slightly lower neutralizing activity as compared to those obtained with F-VLPs. In order to evaluate the persistence of neutralizing antibodies over time, we harvested sera 3 more times (P4, P5, P6), up to 161 days after the first immunization. The dose of 50% inhibition (ID50) was calculated from the neutralizing curves and the results showed a persistence of high levels of neutralizing antibodies (figure 2C).

Altogether, these results indicated that retroviral-derived VLPs incorporating F alone or in combination with G from hMPV C-85473 induce neutralizing antibody responses in mice. Maximal level of neutralizing activity could be reached after 2 consecutive injections.

Induced antibodies are cross-neutralizing between the two different hMPV lineages. Since the F proteins are conserved but are not identical between the two main hMPV lineages, A and B, we sought to compare their antigenicity, immunogenicity and ability to induce cross-protective response.

As schematized in figure 3A, mice were inoculated twice, with 21-day intervals with VLPs incorporating glycoproteins from the lineage A (F_{C-85473}-VLPs and F/G _{C-85473}-VLPs) or from the lineage B (F_{Can98-75}-VLPs). As controls, we inoculated infectious or formalin-inactivated hMPV (hMPV inact). Serum samples (P1 and P2) were harvested 21 days after each vaccination to evaluate the production of antibodies. First, to quantify the total amounts of antibodies against F, we set up an ELISA by coating microplates with F_{C-85473}-VLPs. The results showed that after boost, a high amount of total anti-F antibodies was induced in all the mouse groups. Relative OD was 40- to 60-fold higher for sera from the immunized animals compared to sera from the non-vaccinated mice (figure 3B). Neutralizing antibodies against the F protein from both lineages (F_{C-85473} and F_{Can98-75}) were then determined using the VLP micro-neutralization assay described above (figure 3C and 3D). We chose the 1/100 dilution since we previously demonstrated that it readily discriminated neutralization effect between prime and boost inoculation (figure 2B, C). After the first injection, all vaccinated mice developed neutralizing antibodies against the homologous and heterologous F protein, albeit at different levels. After the boost injection, for most conditions, the neutralization levels reached almost 100%, in agreement with previous results (figure 2).

Then, we compared the results obtained with the micro-neutralization assay to a plaque reduction assay that used both C-85473 and Can98-75 hMPV strains (figure 3E and 3F). hMPV neutralizing-antibody titers were determined by end-point dilution assays. Similar to the VLP-micro-neutralization assay, the results showed that sera from all the mice vaccinated with VLPs displaying glycoproteins from C-85473 or Can98-75 could neutralize both hMPV strains. Of importance, a second vaccination revealed that the neutralization level was higher in the case of F_{C-85473}-VLP vaccinated mice.

Altogether, these results showed that injection of VLPs displaying hMPV glycoproteins from either lineages A or the B induces of a cross-reactive antibody response that can efficiently neutralize hMPV.

Immunization with hMPV-VLP protects mice against hMPV infection. Groups of 18 immunized mice were subsequently challenged intranasally with 1x10⁸ TCID50 of hMPV strain C-85473 or Can98-75 (figure 4A). Six mice per group were weighted daily for 14 days after infection and survival rates were determined (figure 4B and 4C). Usually, hMPV challenge is not lethal in mice (ref); yet, in these conditions, infection by the Can98-75 strain led to 100% mortality by day 7 after challenge for non-immunized mice inoculated with PBS as well as with NoEnv-VLPs (figure 4A and 4B, left panel). In contrast, mice immunized with the homologous virus, Can98-75, recovered and survived the challenge. Similarly, mice immunized with F_{C-85473}-VLPs, F/G _{C-85473}-VLPs or F_{Can98-75}-VLPs survived the challenge (83%, figure 4B), indicating the cross-protection induced by immunization by VLPs harboring non-homologous F or F/G glycoproteins. On the other hand, challenge with the C-85473 strain of hMPV was sublethal (figure 4A and 4B, right panel). Indeed, mice injected with PBS only or with NoEnv-VLPs, exhibited profound weight loss and 33% of them died by day 8 after challenge. In contrast, mice immunized with the homologous virus, C-85473, with homologous or heterologous hMPV-VLPs exhibited partial weight loss and all of them survived the challenge except for one mouse immunized with the heterologous F_{Can98-75}-VLPs.

At 5 days post-challenge, the lungs from 6 mice per group were collected and hMPV present in tissues was quantified by serial dilutions on LLC-MK2 cells. For mice challenged with the C-85473 virus, almost no virus was detected in lungs from animals immunized with homologous virus, with homologous F_{C-85473}-VLPs and F/G _{C-85473}-VLPs or with heterologous F_{Can98-75}-VLP, in contrast to naive mice or to mice injected with NoEnv-VLPs (figure 4D). In the case of Can98-75 challenge, residual virus was detected among all groups of immunized mice, but the amount of virus was significantly lower than that present in control mice (figure 4D).

Altogether, these results showed that hMPV-VLPs inoculation induce cross-protection in mice. hMPV-VLPs induce a Th2 immune response in mice.

### References

- 1.: Akahata, W., et al. Nat Med 16:334-338.
- 2.: Alvarez, R., and R. A. Tripp. 2005. et al. J Virol 79:5971-5978.
- 3.: Bellier, B. et al. 2009. Vaccine 27:5772-5780.
- 4.: Biacchesi, S. et al. 2003. Virology 315:1-9.
- 5.: Boivin, G. et al. 2004. Emerg Infect Dis 10:1154-1157.
- 6.: Cane, P. A. et al. 2003. Bone Marrow Transplant 31:309-310.
- 7.: Chin, J. et al. 1969. Am J Epidemiol 89:449-463.
- 8.: Cseke, G. et al. 2007. J Virol 81:698-707.
- 9.: Dalba, C., et al. 2007. Mol Ther 15:457-466.
- 10.: de Swart, R. L. et al. 2007. Vaccine 25:8518-8528.
- 11.: Delgado, M. F. et al. 2009. Nat Med 15:34-41.
- 12.: Douville, R. N. et al. 2006. J Immunol 176:5848-5855.
- 13.: Frecha, C. et al. 2008. Blood 112:4843-4852.
- 14.: Fulginiti, V. A. et al. 1969. Am J Epidemiol 89:435-448.
- 15.: Garrone, P. et al. 2011. Sci Transl Med 3:94ra71.
- 16.: Hamelin, M. E. et al. 2007. J Gen Virol 88:3391-3400.
- 17.: Hamelin, M. E. et al. 2006. J Infect Dis 193:1634-1642.
- 18.: Hamelin, M. E. et al. 2005. J Virol 79:8894-8903.
- 19.: Harper, D. M. et al 2006. Lancet 367:1247-1255.
- 20.: Hatziioannou, T. et al. 1998. J Virol 72:5313-5317.
- 21.: Herfst, S. et al. 2007. IJ Gen Virol 88:2702-2709.
- 22.: Herfst, S. et al. 2008. Vaccine 26:4224-4230.
- 23.: Kwilas, S. et al. 2009. J Virol 83:10710-10718.
- 24.: Liu, Y. V. et al. 2011. Vaccine 29:6606-6613.
- 25.: Mathieu, C. et al. 2011. J Virol 85:7863-7871.
- 26.: McGinnes, L. W. et al. 2011. J Virol 85:366-377.
- 27.: Mok, H. et al. 2008. J Virol 82:11410-11418.
- 28.: Murawski, M. R. et al. 2010. J Virol 84:1110-1123.
- 29.: Okamoto, M. et al. 2010. J Med Virol 82:2092-2096.
- 30.: Pelletier, G. et al. 2002. Emerg Infect Dis 8:976-978.
- 31.: Peret, T. C. et al. 2004. J Gen Virol 85:679-686.
- 32.: Prince, G. A., et al. 2001. J Gen Virol 82:2881-2888.
- 33.: Quan, F. S. et al. 2011. J Infect Dis 204:987-995.
- 34.: Roy, P., and R. Noad. 2008. Human vaccines 4:5-12.
- 35.: Ryder, A. B. et al. 2010. Vaccine 28:4145-4152.

- 36.: Sandrin, V. et al. 2002. Blood 100:823-832.
- 37.: Skiadopoulos, M. H. et al. 2006. Virology 345:492-501.
- 38.: Skiadopoulos, M. H. et al. 2004. J Virol 78:6927-6937.
- 39.: Szecsi, J., et al. 2006. Virol J 3:70.
- 40.: Szecsi, J. et al. 2009. J Infect Dis 200:181-190.
- 41.: Tang, R. S. et al. 2005. Vaccine 23:1657-1667.
- 42.: Thammawat, S. et al. 2008. J Virol 82:11767-11774.
- 43.: van den Hoogen, B. G. et al. 2002. Virology 295:119-132.
- 44.: van den Hoogen, B. G. et al. 2001. Nat Med 7:719-724.
- 45.: van den Hoogen, B. G. et al. 2007. J Gen Virol 88:1251-1259.
- 46.: Williams, J. V. et al. 2005. J Infect Dis 192:1149-1153.
- 47.: Williams, J. V. et al. 2005. J Infect Dis 192:1061-1065.
- 48.: Yim, K. C. et al. 2007. Vaccine 25:5034-5040.
- 49.: Schowalter R.M. et al. 2006. J. Virol. 80:22, 10931-10941.

## Claims

1. A chimeric virus-like particle, comprising a retrovirus Gag protein, and a human metapneumovirus (hMPV) F protein.

2. The chimeric virus-like particle according to claim 1, wherein the F protein is under the F0 form or under the form of the F1 and F2 subunits.

3. The chimeric virus-like particle according to claim 1 or 2, comprising F protein from sub-group A hMPV, from sub-group B hMPV or from sub-group A hMPV and sub-group B hMPV.

4. The chimeric virus-like particle according to any of claims 1 to 3, comprising a Gag protein of MLV, ALV, RSV, MPMV, HIV-1, HIV-2, SIV, EIAV, CAEV, or HFV, preferably MLV..

5. The chimeric virus-like particle according to any of claims 1 to 4, which further comprises a packaging competent retroviral-derived genome, and possibly said packaging competent retroviral-derived genome further comprises a transgene.

6. An immunogenic or vaccine composition comprising:
- a chimeric virus-like particle according to any of claims 1 to 5 and a pharmaceutically acceptable carrier or vehicle, or
- an expression vector or expression vectors in a pharmaceutically acceptable carrier or vehicle able to produce *in vivo* a chimeric virus-like particle according to any of claims 1 to 5.

7. An expression vector or a set of expression vectors usable in the immunogenic or vaccine composition of claim 6, comprising:
- a first nucleic acid sequence comprising a cDNA encoding a retrovirus Gag protein ;
- a second nucleic acid sequence comprising a cDNA encoding F protein of a human metapneumovirus (hMPV);
- wherein said first and second nucleic acid sequences are contained within the same vector or different vectors;
- and regulatory elements allowing in a human being expression of the cDNAs to produce F from hMPV and the retrovirus Gag protein, and the proteins to form virus-like particles.

8. The expression vector according to claim 7, wherein the second nucleic acid sequence comprises a cDNA encoding a F protein from sub-group A hMPV, from sub-group B hMPV or from sub-group A hMPV and sub-group B hMPV.

9. The expression vector according to claim 7 or 8, comprising a first nucleic acid sequence comprising a cDNA encoding a Gag protein of MLV, ALV, RSV, MPMV, HIV-1, HIV-2, SIV, EIAV, CAEV, or HFV, preferably MLV.

10. A method for *ex vivo* producing a chimeric virus-like particle according to any one of claims 1 to 5, comprising the steps of:
-- providing a first nucleic acid sequence comprising a cDNA encoding a retrovirus Gag protein ;
- providing a second nucleic acid sequence comprising a cDNA encoding F protein of hMPV;
- transfecting host cells with said nucleic acid sequences and maintaining the transfected cells in culture for sufficient time to allow expression of the cDNAs to produce structural proteins from hMPV and retrovirus; and allowing the structural proteins to form virus-like particles,
- possibly treating the obtained virus-like particles with an agent that cleaves F into F1 and F2.

11. A method of *ex vivo* screening or identification of molecules capable of interfering with human metapneumovirus (hMPV) entry in cells comprising comparison of the level of cell infection by an infectious chimeric virus-like particle according to any of claims 1 to 5 in the presence or the absence of a candidate molecule.

12. A method of *in vitro* diagnosis of human metapneumovirus (hMPV) infection in a patient, comprising detecting immune complexes formed by interaction of anti-HMPV antibodies likely to be present in a biological sample of the patient with chimeric virus-like particle according to any of claims 1 to 5.

13. A method of *in vitro* diagnosis human metapneumovirus (hMPV) infection in a patient, comprising detecting an inhibitory effect of anti-hMPV antibodies likely to be present in a biological sample of the patient, on the infection of a permissive cell by chimeric virus-like particle according to any of claims 1 to 5.

14. A transformed host cell that contains :
- a first nucleic acid sequence comprising a cDNA a retrovirus Gag protein;
- a second nucleic acid sequence comprising a cDNA encoding an human metapneumovirus (hMPV) F protein,
- possibly a third nucleic acid sequence comprising a packaging competent retroviral-derived genome.

15. A method of *ex vivo* screening or identification of molecules capable of interfering with human metapneumovirus (hMPV) entry in cells comprising comparing the level of fusion of transformed host cell, as defined in claim 14, to a target host cell, in the presence or the absence of a candidate molecule.


SEQUENCE LISTING

<110>  ECOLE NORMALE SUPERIEURE DE LYON (E.N.S. LYON)
       UNIVERSITE CLAUDE BERNARD DE LYON 1 (U.C.B.L)

<120>  Infectious chimeric pseudo-particles containing functional hMPV
       proteins

<130>  BET12L0540

<160>  16

<170>  PatentIn version 3.5

<210>  1
<211>  61
<212>  DNA
<213>  Artificial Sequence

<220>
<223>  Primer

<400>  1
attcggccgc tcgagcacca tggcttcttg gaaagtggtg atcatttttt cattgttaat      60

a                                                                      61


<210>  2
<211>  48
<212>  DNA
<213>  Artificial Sequence

<220>
<223>  Primer

<400>  2
attcggccga gatctctaat tatgtggtat gaagccattg tttgtgac                   48


<210>  3
<211>  61
<212>  DNA
<213>  Artificial Sequence

<220>
<223>  Primer

<400>  3
attcggccgc tcgagcacca tggcttcttg gaaagtgatg attatcattt cgttactcat      60

a                                                                      61


<210>  4
<211>  48
<212>  DNA
<213>  Artificial Sequence

<220>
<223>  Primer

<400>  4
attcggccga gatctctaac tatgcggtat aaaaccgccg ttggtaac                   48


<210>  5
<211>  54

<212>  DNA
<213>  Artificial Sequence

<220>
<223>  Primer

<400>  5
attcggccgc tcgagcacca tggaggtgaa agtggagaac attcgaacaa taga          54


<210>  6
<211>  49
<212>  DNA
<213>  Artificial Sequence

<220>
<223>  Primer

<400>  6
attcggccga gatctttaac tagtttggtt gtatgttgtt gatgtgctg               49


<210>  7
<211>  54
<212>  DNA
<213>  Artificial Sequence

<220>
<223>  Primer

<400>  7
attcggccgc tcgagcacca tggaagcaag agtggagaac attcgggcaa taga          54


<210>  8
<211>  49
<212>  DNA
<213>  Artificial Sequence

<220>
<223>  Primer

<400>  8
attcggccga gatctttaac tacttggaga agatgtgtct gtgttgtgt               49


<210>  9
<211>  1620
<212>  DNA
<213>  Human metapneumovirus

<400>  9
atgtcttgga aagtggtgat cattttttca ttgttaataa cacctcaaca cggtcttaaa      60

gagagctatt tagaagagtc atgtagcact ataactgaag gatatctcag tgttctgagg     120

acaggttggt ataccaacgt ttttacactg gaggtaggtg atgtagagaa ccttacatgt     180

gctgatggac ctagcttaat aaaaacagaa ttagacctga ccaaaagtgc actaagagaa     240

ctcagaacag tttctgctga tcaactggca agagaggagc aaattgagaa tcccagacaa     300

tctagatttg ttctaggagc aatagcactc ggtgttgcaa cagcagctgc agttacagca     360

ggtgttgcaa ttgccaaaac catccggctt gaaagtgaag taacagcaat taagaatgcc     420

ctcaaaaaga ccaatgaagc agtatctaca ttggggaatg gagttcgagt gttggcaact     480

gcagtgaggg agctggaaga ttttgtgagc aagaatctaa cacgtgcaat caacaaaaac 540

aagtgcgaca ttgctgacct gaaaatggcc gttagcttca gtcaattcaa cagaaggttt 600

ctaaatgttg tgcggcaatt ttcagacaat gctggaataa caccagcaat atccttggac 660

ttaatgacag atgctgaact agccagagct gtttccaaca tgccaacatc tgcaggacaa 720

ataaaactga tgttggagaa ccgtgcaatg gtaagaagaa aggggttcgg aatcctgata 780

ggagtttacg gaagctccgt aatttacatg gtgcaactgc caatctttgg agttatagac 840

acgccttgct ggatagtaaa agcggcccct tcttgctcag aaaaaaaggg aaactatgct 900

tgccttttaa gagaagatca aggatggtat tgtcagaatg cagggtcaac tgtttactac 960

ccaaatgaaa aagactgcga aacaagagga gaccatgtct tttgcgacac agcagcagga 1020

atcaatgttg ctgagcagtc aaaggagtgc aacatcaaca tatccactac taattaccca 1080

tgcaaagtta gcacaggaag acaccctatc agtatggttg cactgtctcc tcttggggct 1140

ttggttgctt gctacaaggg agtgagctgt tccattggca gcaacagagt agggatcatc 1200

aagcaactga acaaaggctg ctcttatata accaaccaag acgcagacac agtgacaata 1260

gacaacactg tataccagct aagcaaagtt gagggcgaac agcatgttat aaaaggaagg 1320

ccagtgtcaa gcagctttga tccagtcaaa tttcctgaag atcaattcaa tgttgcactt 1380

gaccaagttt tcgaaagcat tgagaacagt caggccttgg tggatcaatc aaacagaatc 1440

ctaagcagtg cagagaaagg aaacactggc ttcatcattg taataattct aattgctgtc 1500

cttggctcta ccatgatcct agtgagtgtt tttatcataa taaagaaaac aaagaaaccc 1560

acaggagcac ctccagagct gagtggtgtc acaaacaatg gcttcatacc acataattag 1620

<210> 10
<211> 711
<212> DNA
<213> Human metapneumovirus

<400> 10
atggaggtga aagtggagaa cattcgaaca atagatatgc tcaaagcaag agtgaaaaat 60

cgtgtggcac gcagcaaatg ctttaaaaat gcctctttga tcctaatagg aataactaca 120

ttgagtatag ccctcaatat ctatctaatc ataaactata caatgcaaga aaacacatcc 180

gaatcagaac atcacaccag ctcatcaccc atggaatcca gcagggaaac tccaacagtc 240

cctatggaca actcagacac caatccaggc tcacagtatc caactcaaca gtccacagaa 300

ggctccacac tctactttgc agcctcagca agctcaccag agacagaacc aacatcaaca 360

ccagacacaa caagccgccc gcccttcgtc gacacacaca caacactacc aagtgcaagc 420

agaacaagga caagtccggc agtccacaca aaaaacaatc caaggacaag ccccagaaca 480

cattccccac catgggcaat gacaaggacg gtccgtggaa ccaccactct ccgcacaagc 540

agcacaagaa aaagaccgtc cacagcatca gtccaacctg acagcagcgc aacaacccac 600

aaacacgaag aagcaagccc agtgagcccg caaacatctg cgagcacagc aagaccacaa 660

aggaagggca tggaggccag cacatcaaca acatacaacc aaactagtta a 711

<210>  11
<211>  539
<212>  PRT
<213>  Human metapneumovirus

<400>  11

Met Ser Trp Lys Val Val Ile Ile Phe Ser Leu Leu Ile Thr Pro Gln
1               5                   10                  15

His Gly Leu Lys Glu Ser Tyr Leu Glu Glu Ser Cys Ser Thr Ile Thr
            20                  25                  30

Glu Gly Tyr Leu Ser Val Leu Arg Thr Gly Trp Tyr Thr Asn Val Phe
        35                  40                  45

Thr Leu Glu Val Gly Asp Val Glu Asn Leu Thr Cys Ala Asp Gly Pro
    50                  55                  60

Ser Leu Ile Lys Thr Glu Leu Asp Leu Thr Lys Ser Ala Leu Arg Glu
65                  70                  75                  80

Leu Arg Thr Val Ser Ala Asp Gln Leu Ala Arg Glu Glu Gln Ile Glu
                85                  90                  95

Asn Pro Arg Gln Ser Arg Phe Val Leu Gly Ala Ile Ala Leu Gly Val
            100                 105                 110

Ala Thr Ala Ala Ala Val Thr Ala Gly Val Ala Ile Ala Lys Thr Ile
        115                 120                 125

Arg Leu Glu Ser Glu Val Thr Ala Ile Lys Asn Ala Leu Lys Lys Thr
    130                 135                 140

Asn Glu Ala Val Ser Thr Leu Gly Asn Gly Val Arg Val Leu Ala Thr
145                 150                 155                 160

Ala Val Arg Glu Leu Glu Asp Phe Val Ser Lys Asn Leu Thr Arg Ala
                165                 170                 175

Ile Asn Lys Asn Lys Cys Asp Ile Ala Asp Leu Lys Met Ala Val Ser
            180                 185                 190

Phe Ser Gln Phe Asn Arg Arg Phe Leu Asn Val Val Arg Gln Phe Ser
        195                 200                 205

Asp Asn Ala Gly Ile Thr Pro Ala Ile Ser Leu Asp Leu Met Thr Asp
    210                 215                 220

Ala Glu Leu Ala Arg Ala Val Ser Asn Met Pro Thr Ser Ala Gly Gln
225                 230                 235                 240

Ile Lys Leu Met Leu Glu Asn Arg Ala Met Val Arg Arg Lys Gly Phe
                245                 250                 255

Gly Ile Leu Ile Gly Val Tyr Gly Ser Ser Val Ile Tyr Met Val Gln
            260                 265                 270

Leu Pro Ile Phe Gly Val Ile Asp Thr Pro Cys Trp Ile Val Lys Ala
        275                 280                 285

Ala Pro Ser Cys Ser Glu Lys Lys Gly Asn Tyr Ala Cys Leu Leu Arg
    290                 295                 300

Glu Asp Gln Gly Trp Tyr Cys Gln Asn Ala Gly Ser Thr Val Tyr Tyr
305                 310                 315                 320

Pro Asn Glu Lys Asp Cys Glu Thr Arg Gly Asp His Val Phe Cys Asp
                325                 330                 335

Thr Ala Ala Gly Ile Asn Val Ala Glu Gln Ser Lys Glu Cys Asn Ile
            340                 345                 350

Asn Ile Ser Thr Thr Asn Tyr Pro Cys Lys Val Ser Thr Gly Arg His
        355                 360                 365

Pro Ile Ser Met Val Ala Leu Ser Pro Leu Gly Ala Leu Val Ala Cys
    370                 375                 380

Tyr Lys Gly Val Ser Cys Ser Ile Gly Ser Asn Arg Val Gly Ile Ile
385                 390                 395                 400

Lys Gln Leu Asn Lys Gly Cys Ser Tyr Ile Thr Asn Gln Asp Ala Asp
                405                 410                 415

Thr Val Thr Ile Asp Asn Thr Val Tyr Gln Leu Ser Lys Val Glu Gly
            420                 425                 430

Glu Gln His Val Ile Lys Gly Arg Pro Val Ser Ser Ser Phe Asp Pro
        435                 440                 445

Val Lys Phe Pro Glu Asp Gln Phe Asn Val Ala Leu Asp Gln Val Phe
    450                 455                 460

Glu Ser Ile Glu Asn Ser Gln Ala Leu Val Asp Gln Ser Asn Arg Ile
465                 470                 475                 480

Leu Ser Ser Ala Glu Lys Gly Asn Thr Gly Phe Ile Ile Val Ile Ile
                485                 490                 495

Leu Ile Ala Val Leu Gly Ser Thr Met Ile Leu Val Ser Val Phe Ile
            500                 505                 510

Ile Ile Lys Lys Thr Lys Lys Pro Thr Gly Ala Pro Pro Glu Leu Ser
        515                 520                 525

Gly Val Thr Asn Asn Gly Phe Ile Pro His Asn
    530                 535

<210>  12
<211>  236
<212>  PRT
<213>  Human metapneumovirus

<400>  12

Met Glu Val Lys Val Glu Asn Ile Arg Thr Ile Asp Met Leu Lys Ala
1               5                   10                  15

Arg Val Lys Asn Arg Val Ala Arg Ser Lys Cys Phe Lys Asn Ala Ser
            20                  25                  30

Leu Ile Leu Ile Gly Ile Thr Thr Leu Ser Ile Ala Leu Asn Ile Tyr
        35                  40                  45

Leu Ile Ile Asn Tyr Thr Met Gln Glu Asn Thr Ser Glu Ser Glu His
    50                  55                  60

His Thr Ser Ser Ser Pro Met Glu Ser Ser Arg Glu Thr Pro Thr Val
65                  70                  75                  80

Pro Met Asp Asn Ser Asp Thr Asn Pro Gly Ser Gln Tyr Pro Thr Gln
                85                  90                  95

Gln Ser Thr Glu Gly Ser Thr Leu Tyr Phe Ala Ala Ser Ala Ser Ser
            100                 105                 110

Pro Glu Thr Glu Pro Thr Ser Thr Pro Asp Thr Thr Ser Arg Pro Pro
        115                 120                 125

Phe Val Asp Thr His Thr Thr Leu Pro Ser Ala Ser Arg Thr Arg Thr
    130                 135                 140

Ser Pro Ala Val His Thr Lys Asn Asn Pro Arg Thr Ser Pro Arg Thr
145                 150                 155                 160

His Ser Pro Pro Trp Ala Met Thr Arg Thr Val Arg Gly Thr Thr Thr
                165                 170                 175

Leu Arg Thr Ser Ser Thr Arg Lys Arg Pro Ser Thr Ala Ser Val Gln
            180                 185                 190

Pro Asp Ser Ser Ala Thr Thr His Lys His Glu Glu Ala Ser Pro Val
        195                 200                 205

Ser Pro Gln Thr Ser Ala Ser Thr Ala Arg Pro Gln Arg Lys Gly Met
    210                 215                 220

Glu Ala Ser Thr Ser Thr Thr Tyr Asn Gln Thr Ser
225                 230                 235

<210>  13
<211>  1645
<212>  DNA
<213>  Human metapneumovirus

<400>  13
gggacaagta aaaatgtctt ggaaagtgat gattatcatt tcgttactca taacacctca      60
gcacggacta aaggaaagtt atttagaaga atcatgtagt actataactg aaggatatct     120
cagtgtttta agaacaggtt ggtacaccaa tgtctttaca ttagaagttg gtgatgttga     180
aaatcttaca tgtactgatg gacctagctt aatcaaaaca gaacttgacc taaccaaaag     240
tgctctaaga gaactcaaaa cagtttctgc tgatcagtta gcgagagaag aacaaattga     300
aaatcccaga caatcaaggt ttgtcctagg tgcaatagct cttggtgttg ccacagcagc     360
agcagtcaca gcaggcattg cgatagccaa aaccataagg cttgagagtg aagtgaatgc     420
aatcaaaggt gctctcaaaa caaccaatga ggcagtatcc acactaggaa atggagtgcg     480
agtcctagcc accgcagtaa gagagctgaa agaatttgtg agcaaaaacc tgactagtgc     540
aattaacaag aacaaatgtg acattgctga tctgaagatg gctgtcagct tcagtcaatt     600
caacagaaga ttcctaaatg ttgtgcggca gttttcagac aatgcaggga taacaccagc     660
aatatcattg gacctaatga ctgatgctga gctggccaga gctgtatcat acatgccaac     720
atctgcagga cagataaaac taatgttaga gaaccgtgca atggtgagga gaaaaggatt     780
tggaatcttg ataggggtct acggaagctc cgtgatttac atggtccagc tgccgatctt     840
tggtgtcata gatacacctt gttggataat caaggcagct ccctcttgtt cagaaaaaga     900
tggaaactat gcttgcctcc taagagagga tcaagggtgg tattgtaaaa atgcaggatc     960
cactgtttac tacccaaata aaaaagactg cgaaacaaga ggtgatcatg ttttttgtga    1020
cacagctgca gggatcaatg ttgctgagca atcaagagaa tgcaacatca acatatctac    1080
aaccaactac ccatgcaaag tcagcacagg aagacaccct atcagcatgg ttgcactatc    1140
acctctcggt gctttggtgg cttgctacaa aggggttagc tgttcaattg gcagtaatcg    1200
ggttggaata atcaaacaac tacctaaagg ctgctcatac ataactaacc aggacgcaga    1260
cactgtaaca attgacaaca ctgtgtatca actaagcaaa gttgagggtg aacagcatgt    1320
aataaaaggg agaccagttt caagcagttt cgatccaatc aagtttcctg aggatcagtt    1380
caatgttgcg cttgatcaag tctttgaaag cattgaaaac agtcaagcac tagtggacca    1440
gtcaaacaaa attctgaaca gtgcagaaaa aggaaacact ggcttcatta ttgtaataat    1500
tttgattgct gttcttgggt taaccatgat ttcagtgagc atcatcatca taatcaaaaa    1560
aacaaggaaa cccacagggg cacctccaga gctgaatggt gttaccaacg gcggttttat    1620

accgcatagt tagttaatta aaaaa                                          1645

<210>  14
<211>  732
<212>  DNA
<213>  Human metapneumovirus

<400>  14
gggacaagtg gccatggaag caagagtgga gaacattcgg gcaatagaca tgttcaaagc      60

aaagatgaaa aaccgtataa gaagtagcaa gtgccataga aatgctacac tgatccttat     120

tggatcaaca gcaccaagta tggcactcaa caccctttta atcattgatc atgcaacatc     180

aaaaaacatg accaaagtgg aacactgtgt caacatgccg ccggtagaac caagcaagaa     240

gaccccaatg acctctgcag cagacccaaa caccaaaccc aatccacagc aggcaacaca     300

gctgaccaca gaggattcaa catctctagc agcaacccta gaggaccatc tacacacagg     360

gacaactcca acaccagatg caacagtctc ccagcaaacc acagacgagc acacaacact     420

gctgagatca accaacagac agaccaccca aacaaccgca gagaaaaagc caaccagagc     480

aacaaccaaa aaagaaacca caactcgaac cacaagcaca gctgcaaccc aaacactcaa     540

caccaccaac caaactagca atggaagaga ggcaaccaca acatctgcca gatccagaaa     600

caatgccaca actcaaagca gcgatcaaac aacccaggca gcagacccaa gctcccaatc     660

acaacataca cagaaaagca caacaacaac acacaacaca gacacatctt ctccaagtag     720

ttaacaaaaa aa                                                         732

<210>  15
<211>  539
<212>  PRT
<213>  Human metapneumovirus

<400>  15

Met Ser Trp Lys Val Met Ile Ile Ile Ser Leu Leu Ile Thr Pro Gln
1               5                   10                  15

His Gly Leu Lys Glu Ser Tyr Leu Glu Glu Ser Cys Ser Thr Ile Thr
            20                  25                  30

Glu Gly Tyr Leu Ser Val Leu Arg Thr Gly Trp Tyr Thr Asn Val Phe
        35                  40                  45

Thr Leu Glu Val Gly Asp Val Glu Asn Leu Thr Cys Thr Asp Gly Pro
    50                  55                  60

Ser Leu Ile Lys Thr Glu Leu Asp Leu Thr Lys Ser Ala Leu Arg Glu
65                  70                  75                  80

Leu Lys Thr Val Ser Ala Asp Gln Leu Ala Arg Glu Glu Gln Ile Glu
                85                  90                  95

Asn Pro Arg Gln Ser Arg Phe Val Leu Gly Ala Ile Ala Leu Gly Val

            100                 105                 110

Ala Thr Ala Ala Ala Val Thr Ala Gly Ile Ala Ile Ala Lys Thr Ile
        115                 120                 125

Arg Leu Glu Ser Glu Val Asn Ala Ile Lys Gly Ala Leu Lys Thr Thr
    130                 135                 140

Asn Glu Ala Val Ser Thr Leu Gly Asn Gly Val Arg Val Leu Ala Thr
145                 150                 155                 160

Ala Val Arg Glu Leu Lys Glu Phe Val Ser Lys Asn Leu Thr Ser Ala
                165                 170                 175

Ile Asn Lys Asn Lys Cys Asp Ile Ala Asp Leu Lys Met Ala Val Ser
            180                 185                 190

Phe Ser Gln Phe Asn Arg Arg Phe Leu Asn Val Val Arg Gln Phe Ser
        195                 200                 205

Asp Asn Ala Gly Ile Thr Pro Ala Ile Ser Leu Asp Leu Met Thr Asp
    210                 215                 220

Ala Glu Leu Ala Arg Ala Val Ser Tyr Met Pro Thr Ser Ala Gly Gln
225                 230                 235                 240

Ile Lys Leu Met Leu Glu Asn Arg Ala Met Val Arg Arg Lys Gly Phe
                245                 250                 255

Gly Ile Leu Ile Gly Val Tyr Gly Ser Ser Val Ile Tyr Met Val Gln
            260                 265                 270

Leu Pro Ile Phe Gly Val Ile Asp Thr Pro Cys Trp Ile Ile Lys Ala
        275                 280                 285

Ala Pro Ser Cys Ser Glu Lys Asp Gly Asn Tyr Ala Cys Leu Leu Arg
    290                 295                 300

Glu Asp Gln Gly Trp Tyr Cys Lys Asn Ala Gly Ser Thr Val Tyr Tyr
305                 310                 315                 320

Pro Asn Lys Lys Asp Cys Glu Thr Arg Gly Asp His Val Phe Cys Asp
                325                 330                 335

Thr Ala Ala Gly Ile Asn Val Ala Glu Gln Ser Arg Glu Cys Asn Ile
            340                 345                 350

Asn Ile Ser Thr Thr Asn Tyr Pro Cys Lys Val Ser Thr Gly Arg His
        355                 360                 365

Pro Ile Ser Met Val Ala Leu Ser Pro Leu Gly Ala Leu Val Ala Cys

370                 375                 380

Tyr Lys Gly Val Ser Cys Ser Ile Gly Ser Asn Arg Val Gly Ile Ile
385                 390                 395                 400

Lys Gln Leu Pro Lys Gly Cys Ser Tyr Ile Thr Asn Gln Asp Ala Asp
                405                 410                 415

Thr Val Thr Ile Asp Asn Thr Val Tyr Gln Leu Ser Lys Val Glu Gly
            420                 425                 430

Glu Gln His Val Ile Lys Gly Arg Pro Val Ser Ser Ser Phe Asp Pro
        435                 440                 445

Ile Lys Phe Pro Glu Asp Gln Phe Asn Val Ala Leu Asp Gln Val Phe
    450                 455                 460

Glu Ser Ile Glu Asn Ser Gln Ala Leu Val Asp Gln Ser Asn Lys Ile
465                 470                 475                 480

Leu Asn Ser Ala Glu Lys Gly Asn Thr Gly Phe Ile Ile Val Ile Ile
                485                 490                 495

Leu Ile Ala Val Leu Gly Leu Thr Met Ile Ser Val Ser Ile Ile Ile
            500                 505                 510

Ile Ile Lys Lys Thr Arg Lys Pro Thr Gly Ala Pro Pro Glu Leu Asn
        515                 520                 525

Gly Val Thr Asn Gly Gly Phe Ile Pro His Ser
    530                 535

<210>  16
<211>  236
<212>  PRT
<213>  Human metapneumovirus

<400>  16

Met Glu Ala Arg Val Glu Asn Ile Arg Ala Ile Asp Met Phe Lys Ala
1               5                   10                  15

Lys Met Lys Asn Arg Ile Arg Ser Ser Lys Cys His Arg Asn Ala Thr
            20                  25                  30

Leu Ile Leu Ile Gly Ser Thr Ala Pro Ser Met Ala Leu Asn Thr Leu
        35                  40                  45

Leu Ile Ile Asp His Ala Thr Ser Lys Asn Met Thr Lys Val Glu His
    50                  55                  60

Cys Val Asn Met Pro Pro Val Glu Pro Ser Lys Lys Thr Pro Met Thr
65                  70                  75                  80

Ser Ala Ala Asp Pro Asn Thr Lys Pro Asn Pro Gln Gln Ala Thr Gln
                85                  90                  95

Leu Thr Thr Glu Asp Ser Thr Ser Leu Ala Ala Thr Leu Glu Asp His
            100                 105                 110

Leu His Thr Gly Thr Thr Pro Thr Pro Asp Ala Thr Val Ser Gln Gln
        115                 120                 125

Thr Thr Asp Glu His Thr Thr Leu Leu Arg Ser Thr Asn Arg Gln Thr
    130                 135                 140

Thr Gln Thr Thr Ala Glu Lys Lys Pro Thr Arg Ala Thr Thr Lys Lys
145                 150                 155                 160

Glu Thr Thr Thr Arg Thr Thr Ser Thr Ala Ala Thr Gln Thr Leu Asn
                165                 170                 175

Thr Thr Asn Gln Thr Ser Asn Gly Arg Glu Ala Thr Thr Thr Ser Ala
            180                 185                 190

Arg Ser Arg Asn Asn Ala Thr Thr Gln Ser Ser Asp Gln Thr Thr Gln
        195                 200                 205

Ala Ala Asp Pro Ser Ser Gln Ser Gln His Thr Gln Lys Ser Thr Thr
    210                 215                 220

Thr Thr His Asn Thr Asp Thr Ser Ser Pro Ser Ser
225                 230                 235